# EUROPEAN PATENT APPLICATION

(11) **EP 4 825 872 A1**
(43) Date of publication of application: **21.10.2026**
(21) Application number: 24902862.2
(22) Date of filing: 11.12.2024
(51) Int. Cl.: C07K 16/18, C07K 16/40, A61K 39/395, A61P 35/04, A61K 47/68

(54) **ADAM9 BINDING PROTEIN AND USE THEREOF**

(30) Priority: 14.12.2023 CN 202311734757; 06.02.2024 CN 202410173238; 06.12.2024 CN 202411789351
(71) Applicant: Sichuan Kelun-Biotech Biopharmaceutical Co., Ltd., Chengdu, Sichuan 611138 (CN)
(72) Inventor: HU, Jiangjiang, Chengdu, Sichuan 611138 (CN); CHEN, Mei, Chengdu, Sichuan 611138 (CN); LIU, Hanjie, Chengdu, Sichuan 611138 (CN); JIANG, Hongjiao, Chengdu, Sichuan 611138 (CN); YANG, Yang, Chengdu, Sichuan 611138 (CN); LONG, Hu, Chengdu, Sichuan 611138 (CN); TAN, Miao, Chengdu, Sichuan 611138 (CN); TAN, Xiangyang, Chengdu, Sichuan 611138 (CN); GE, Junyou, Chengdu, Sichuan 611138 (CN)
(74) Representative: Santarelli
(86) International application number: PCT/CN2024/138563
(87) International publication number: WO 2025/124443

(57) **Abstract**

The present invention relates to the field of disease treatment. Provided are an anti-ADAM9 antibody or an antigen-binding fragment thereof, a nucleic acid molecule encoding same, and a method for preparing same. The anti-ADAM9 antibody or the antigen-binding fragment thereof has high specificity and high affinity for ADAM9. The present invention further relates to the use of the antibody or the antigen-binding fragment thereof in the treatment and diagnosis of a disease.

## Description

### Cross-Reference to Related Applications

The present application claims priority to Chinese Patent Application No. 202311734757.9 filed on December 14, 2023, Chinese Patent Application No. 202410173238.8 filed on February 6, 2024, and to Chinese Patent Application No. 202411789351.5 filed on December 6, 2024, the contents of which are incorporated herein by reference in their entirety.

### Technical Field

The present disclosure relates to the field of therapeutic monoclonal antibodies. More particularly, the present disclosure relates to an antibody against ADAM9. The present disclosure also relates to the use of the antibody in the treatment and diagnosis of a disease.

### Background

A disintegrin and a metalloprotease (ADAM) are type I transmembrane proteins anchored to the cell surface membrane. More than 30 types have been discovered so far. Similar to matrix metalloproteinases (MMPs), ADAMs also include a prodomain and a zinc-binding metalloproteinase domain. In addition, ADAMs include a disintegrin domain that is unique among cell surface proteins. ADAM9 is widely expressed in humans, regulates a variety of biological functions, and plays an important role in various diseases, including neurodegenerative diseases, retinal diseases, inflammation and tumors. ADAM9 is involved in a variety of physiological functions mainly through the protein-degrading domain for adhesion and the metalloprotein domain for shedding a variety of cell surface proteins.

In recent years, there has been growing evidence that ADAM9 plays an important role in tumors. ADAM9 is involved in the regulation of various tumor processes. In addition to metastasis, ADAM9 plays an important role in tumor proliferation, angiogenesis, and even immune escape. Overexpression of ADAM9 has been found in a variety of cancers and is associated with tumor invasiveness and poor prognosis. In addition, ADAM9 promotes tumor progression, therapeutic resistance and cancer metastasis through proteolytic or non-proteolytic pathways.

A number of studies have shown that overexpression of ADAM9 shortens overall survival. Additional studies have shown that ADAM9 plays a major role in several steps of lung cancer metastasis. Shintani et al. first described that overexpression of ADAM9 promotes tumor cell adhesion to vascular endothelial cells, suggesting the importance of ADAM9 in the metastasis process. In addition, ADAM9 enhances cell migration and stress resistance through a new mechanism to promote metastasis. Studies by Fritzsche et al. have shown that overexpression of ADAM9 mRNA and protein is associated with low recurrence-free survival of prostate cancer. Immunohistochemical analysis showed that the expression of ADAM9 protein was increased in 60% or more of recurrent prostate tumors. IL-6 is a key mediator of liver cancer invasion and metastasis, and IL-6 enhances ADAM9 expression *in vitro* by activating the JNK pathway. Silencing ADAM9 can not only reduce the size of primary tumors but also inhibit the rate of metastasis to the lungs. In contrast, overexpression of ADAM9 accelerates the growth of primary tumors and promotes metastasis to the lungs. ADAM9 shares the methyltransferase with EGFR. Nuclear receptor-binding SET domain protein 2 is a member of the histone methyltransferase family, which can upregulate the expression of ADAM9 and EGFR, thereby promoting the resistance of TNBC cells to EGFR inhibitors. KRAS signaling is essential for maintaining tumorigenesis in pancreatic cancer. Yuan et al. have found that dysregulated KRAS signaling enhances the expression of ADAM9 through the NF-kB cascade, and knockout of ADAM9 inhibits the downstream pathways of KRAS and MEK-ERK signaling. In addition, studies have shown that circular ADAM9 (circ-ADAM9) is upregulated in pancreatic cancer cells, which is associated with poor prognosis. Overexpression of circ-ADAM9 increases ERK signaling, and promotes cell proliferation and migration *in vitro*, while silencing circ-ADAM9 delays the growth of pancreatic tumors *in vivo*.

Studies conducted so far have sufficiently supported the use of ADAM9 as a prognostic marker for cancer. Currently used therapeutic drugs (e.g., Sorafenib and Regorafenib) enhance cancer treatment by reducing ADAM9 levels, which provides new ideas for ADAM9-targeted therapy. At the AACR conference in 2021, ImmunoGen presented the research progress of IMGC936. IMGC936 is the first ADAM9-targeted antibody-drug conjugate (ADC) co-developed by ImmunoGen and Macrogenics. IMGC936 is an innovative drug composed of a high-affinity humanized monoclonal antibody, a maytansinoid microtubule inhibitor payload, and a stable tripeptide linker, with a drug-to-antibody ratio (DAR) of 2, and is currently in the phase I clinical trial stage.

In terms of safety, no major developmental defects were observed in ADAM9 gene-knockout mice, except for subsequent retinal disorders. This suggests that it may be a relatively safe target in adult patients.

Nonetheless, the specificity of therapeutic agents for ADAM9 should be considered when treating ADAM9-related diseases, as regulating the protective activity of MMPs generally causes side effects. Therefore, it is necessary to develop antibodies targeting ADAM9, which will provide patients with more choices of medication.

### Summary of the Invention

In the present application, the inventors have developed a high-affinity humanized antibody with excellent properties, which is capable of specifically recognizing/binding to ADAM9 and can be used for the prevention and/or treatment of cancers that express ADAM9, particularly cancers that overexpress ADAM9.

### Antibodies of the present invention

In one aspect, the present disclosure provides an antibody or an antigen-binding fragment thereof specifically binding to ADAM9, wherein the antibody or the antigen-binding fragment thereof comprises complementarity determining regions (CDRs) as follows:
(a) CDR-H1, CDR-H2 and CDR-H3 contained in a heavy chain variable region (VH) set forth in SEQ ID NO: 54 or 52; and/or, CDR-L1, CDR-L2 and CDR-L3 contained in a light chain variable region (VL) set forth in SEQ ID NO: 55 or 53;
(b) CDR-H1, CDR-H2 and CDR-H3 contained in a heavy chain variable region (VH) set forth in SEQ ID NO: 3 or 1; and/or, CDR-L1, CDR-L2 and CDR-L3 contained in a light chain variable region (VL) set forth in SEQ ID NO: 4 or 2;
(c) CDR-H1, CDR-H2 and CDR-H3 contained in a heavy chain variable region (VH) set forth in SEQ ID NO: 9 or 7; and/or, CDR-L1, CDR-L2 and CDR-L3 contained in a light chain variable region (VL) set forth in SEQ ID NO: 10 or 8;
(d) CDR-H1, CDR-H2 and CDR-H3 contained in a heavy chain variable region (VH) set forth in SEQ ID NO: 50 or 48; and/or, CDR-L1, CDR-L2 and CDR-L3 contained in a light chain variable region (VL) set forth in SEQ ID NO: 51 or 49; or
(e) CDR-H1, CDR-H2 and CDR-H3 contained in a heavy chain variable region (VH) described below, and/or CDR-L1, CDR-L2 and CDR-L3 contained in a light chain variable region (VL) described below, wherein the heavy chain variable region (VH) and/or the light chain variable region (VL) comprises a mutation in at least one CDR compared to the heavy chain variable region and/or light chain variable region of any one of (a) to (d), and the mutation is a substitution, deletion or addition of one or several amino acids (e.g., a substitution, deletion or addition of 1, 2 or 3 amino acids).

In certain embodiments, the antibody or the antigen-binding fragment thereof comprises complementarity determining regions (CDRs) as follows:
(i) CDR-H1, CDR-H2 and CDR-H3 contained in a heavy chain variable region (VH) set forth in SEQ ID NO: 54; and/or, CDR-L1, CDR-L2 and CDR-L3 contained in a light chain variable region (VL) set forth in SEQ ID NO: 55;
(ii) CDR-H1, CDR-H2 and CDR-H3 contained in a heavy chain variable region (VH) set forth in SEQ ID NO: 52; and/or, CDR-L1, CDR-L2 and CDR-L3 contained in a light chain variable region (VL) set forth in SEQ ID NO: 53;
(iii) CDR-H1, CDR-H2 and CDR-H3 contained in a heavy chain variable region (VH) set forth in SEQ ID NO: 3; and/or, CDR-L1, CDR-L2 and CDR-L3 contained in a light chain variable region (VL) set forth in SEQ ID NO: 4;
(iv) CDR-H1, CDR-H2 and CDR-H3 contained in a heavy chain variable region (VH) set forth in SEQ ID NO: 1; and/or, CDR-L1, CDR-L2 and CDR-L3 contained in a light chain variable region (VL) set forth in SEQ ID NO: 2;
(v) CDR-H1, CDR-H2 and CDR-H3 contained in a heavy chain variable region (VH) set forth in SEQ ID NO: 9; and/or, CDR-L1, CDR-L2 and CDR-L3 contained in a light chain variable region (VL) set forth in SEQ ID NO: 10;
(vi) CDR-H1, CDR-H2 and CDR-H3 contained in a heavy chain variable region (VH) set forth in SEQ ID NO: 7; and/or, CDR-L1, CDR-L2 and CDR-L3 contained in a light chain variable region (VL) set forth in SEQ ID NO: 8;
(vii) CDR-H1, CDR-H2 and CDR-H3 contained in a heavy chain variable region (VH) set forth in SEQ ID NO: 50; and/or, CDR-L1, CDR-L2 and CDR-L3 contained in a light chain variable region (VL) set forth in SEQ ID NO: 51;
(viii) CDR-H1, CDR-H2 and CDR-H3 contained in a heavy chain variable region (VH) set forth in SEQ ID NO: 48; and/or, CDR-L1, CDR-L2 and CDR-L3 contained in a light chain variable region (VL) set forth in SEQ ID NO: 49; or
(ix) CDR-H1, CDR-H2 and CDR-H3 contained in a heavy chain variable region (VH) described below, and/or CDR-L1, CDR-L2 and CDR-L3 contained in a light chain variable region (VL) described below, wherein the heavy chain variable region (VH) and/or the light chain variable region (VL) comprises a mutation in at least one CDR compared to the heavy chain variable region and/or light chain variable region of any one of (i) or (iv), and the mutation is a substitution, deletion or addition of one or several amino acids (e.g., a substitution, deletion or addition of 1, 2 or 3 amino acids).

In certain embodiments, the substitution is a conservative substitution.

In certain embodiments, the CDRs are defined according to the IMGT, Kabat, Chothia, or AbM numbering system.

In certain embodiments, the ADAM9 includes human ADAM9 and/or monkey ADAM9. In certain embodiments, the monkey is Macaca mulatta.

In certain embodiments, the antibody or the antigen-binding fragment thereof of the present disclosure comprises a heavy chain variable region (VH) and/or a light chain variable region (VL), wherein CDRs are defined by the IMGT numbering system:
(1a) a heavy chain variable region (VH) comprising 3 CDRs as follows: CDR-H1 having a sequence of SEQ ID NO: 81 or a variant thereof; CDR-H2 having a sequence of SEQ ID NO: 82 or a variant thereof;
and CDR-H3 having a sequence of SEQ ID NO: 83 or a variant thereof; and/or, a light chain variable region (VL) comprising 3 CDRs as follows: CDR-L1 having a sequence of SEQ ID NO: 84 or a variant thereof; CDR-L2 having a sequence of SEQ ID NO: 85 or a variant thereof; and CDR-L3 having a sequence of SEQ ID NO: 76 or a variant thereof; and
(1b-i) a heavy chain variable region (VH) comprising 3 CDRs as follows: CDR-H1 having a sequence of SEQ ID NO: 21 or a variant thereof; CDR-H2 having a sequence of SEQ ID NO: 22 or a variant thereof;
and CDR-H3 having a sequence of SEQ ID NO: 23 or a variant thereof; and/or, a light chain variable region (VL) comprising 3 CDRs as follows: CDR-L1 having a sequence of SEQ ID NO: 24 or a variant thereof; CDR-L2 having a sequence of SEQ ID NO: 25 or a variant thereof; and CDR-L3 having a sequence of SEQ ID NO: 16 or a variant thereof; and
(1b-ii) a heavy chain variable region (VH) comprising 3 CDRs as follows: CDR-H1 having a sequence of SEQ ID NO: 21 or a variant thereof; CDR-H2 having a sequence of SEQ ID NO: 22 or a variant thereof;
and CDR-H3 having a sequence of SEQ ID NO: 94 or a variant thereof; and/or, a light chain variable region (VL) comprising 3 CDRs as follows: CDR-L1 having a sequence of SEQ ID NO: 24 or a variant thereof; CDR-L2 having a sequence of SEQ ID NO: 25 or a variant thereof; and CDR-L3 having a sequence of SEQ ID NO: 16 or a variant thereof; and
(1c-i) a heavy chain variable region (VH) comprising 3 CDRs as follows: CDR-H1 having a sequence of SEQ ID NO: 35 or a variant thereof; CDR-H2 having a sequence of SEQ ID NO: 36 or a variant thereof;
and CDR-H3 having a sequence of SEQ ID NO: 37 or a variant thereof; and/or, a light chain variable region (VL) comprising 3 CDRs as follows: CDR-L1 having a sequence of SEQ ID NO: 38 or a variant thereof; CDR-L2 having a sequence of SEQ ID NO: 39 or a variant thereof; and CDR-L3 having a sequence of SEQ ID NO: 30 or a variant thereof; and
(1c-ii) a heavy chain variable region (VH) comprising 3 CDRs as follows: CDR-H1 having a sequence of SEQ ID NO: 103 or a variant thereof; CDR-H2 having a sequence of SEQ ID NO: 104 or a variant thereof; and CDR-H3 having a sequence of SEQ ID NO: 37 or a variant thereof; and/or, a light chain variable region (VL) comprising 3 CDRs as follows: CDR-L1 having a sequence of SEQ ID NO: 105 or a variant thereof; CDR-L2 having a sequence of SEQ ID NO: 39 or a variant thereof; and CDR-L3 having a sequence of SEQ ID NO: 30 or a variant thereof; or
(1d) a heavy chain variable region (VH) comprising 3 CDRs as follows: CDR-H1 having a sequence of SEQ ID NO: 65 or a variant thereof; CDR-H2 having a sequence of SEQ ID NO: 66 or a variant thereof; and CDR-H3 having a sequence of SEQ ID NO: 67 or a variant thereof; and/or, a light chain variable region (VL) comprising 3 CDRs as follows: CDR-L1 having a sequence of SEQ ID NO: 68 or a variant thereof; CDR-L2 having a sequence of SEQ ID NO: 69 or a variant thereof; and CDR-L3 having a sequence of SEQ ID NO: 70 or a variant thereof; and
wherein the variant in any one of (1a), (1b-i), (1b-ii), (1c-i), (1c-ii) and (1d) has a substitution, deletion or addition of one or several amino acids (e.g., a substitution, deletion or addition of 1, 2 or 3 amino acids) compared to the sequence from which the variant is derived. In certain embodiments, the substitution is a conservative substitution.

In certain embodiments, the antibody or the antigen-binding fragment thereof of the present disclosure comprises a heavy chain variable region (VH) and/or a light chain variable region (VL), wherein CDRs are defined by the Chothia numbering system:
(2a) a heavy chain variable region (VH) comprising 3 CDRs as follows: CDR-H1 having a sequence of SEQ ID NO: 71 or a variant thereof; CDR-H2 having a sequence of SEQ ID NO: 72 or a variant thereof; and CDR-H3 having a sequence of SEQ ID NO: 73 or a variant thereof; and/or, a light chain variable region (VL) comprising 3 CDRs as follows: CDR-L1 having a sequence of SEQ ID NO: 74 or a variant thereof; CDR-L2 having a sequence of SEQ ID NO: 75 or a variant thereof; CDR-L3 having a sequence of SEQ ID NO: 76 or a variant thereof; and
(2b) a heavy chain variable region (VH) comprising 3 CDRs as follows: CDR-H1 having a sequence of SEQ ID NO: 11 or a variant thereof; CDR-H2 having a sequence of SEQ ID NO: 12 or a variant thereof; and CDR-H3 having a sequence of SEQ ID NO: 13 or a variant thereof; and/or, a light chain variable region (VL) comprising 3 CDRs as follows: CDR-L1 having a sequence of SEQ ID NO: 14 or a variant thereof; CDR-L2 having a sequence of SEQ ID NO: 15 or a variant thereof; CDR-L3 having a sequence of SEQ ID NO: 16 or a variant thereof; and
(2c-i) a heavy chain variable region (VH) comprising 3 CDRs as follows: CDR-H1 having a sequence of SEQ ID NO: 11 or a variant thereof; CDR-H2 having a sequence of SEQ ID NO: 26 or a variant thereof; and CDR-H3 having a sequence of SEQ ID NO: 27 or a variant thereof; and/or, a light chain variable region (VL) comprising 3 CDRs as follows: CDR-L1 having a sequence of SEQ ID NO: 28 or a variant thereof; CDR-L2 having a sequence of SEQ ID NO: 29 or a variant thereof; CDR-L3 having a sequence of SEQ ID NO: 30 or a variant thereof;
(2c-ii) a heavy chain variable region (VH) comprising 3 CDRs as follows: CDR-H1 having a sequence of SEQ ID NO: 95 or a variant thereof; CDR-H2 having a sequence of SEQ ID NO: 96 or a variant thereof; CDR-H3 having a sequence of SEQ ID NO: 27 or a variant thereof; and/or, a light chain variable region (VL) comprising 3 CDRs as follows: CDR-L1 having a sequence of SEQ ID NO: 97 or a variant thereof; CDR-L2 having a sequence of SEQ ID NO: 98 or a variant thereof; CDR-L3 having a sequence of SEQ ID NO: 30 or a variant thereof; or
(2d) a heavy chain variable region (VH) comprising 3 CDRs as follows: CDR-H1 having a sequence of SEQ ID NO: 56 or a variant thereof; CDR-H2 having a sequence of SEQ ID NO: 57 or a variant thereof; CDR-H3 having a sequence of SEQ ID NO: 58 or a variant thereof; and/or, a light chain variable region (VL) comprising 3 CDRs as follows: CDR-L1 having a sequence of SEQ ID NO: 59 or a variant thereof; CDR-L2 having a sequence of SEQ ID NO: 60 or a variant thereof; CDR-L3 having a sequence of SEQ ID NO: 61 or a variant thereof;
wherein the variant in any one of (2a), (2b), (2c-i), (2c-ii) and (2d) has a substitution, deletion or addition of one or several amino acids (e.g., a substitution, deletion or addition of 1, 2 or 3 amino acids) compared to the sequence from which the variant is derived. In certain embodiments, the substitution is a conservative substitution.

In certain embodiments, the antibody or the antigen-binding fragment thereof of the present disclosure comprises a heavy chain variable region (VH) and/or a light chain variable region (VL), wherein CDRs are defined by the Kabat numbering system:
(3a) a heavy chain variable region (VH) comprising 3 CDRs as follows: CDR-H1 having a sequence of SEQ ID NO: 79 or a variant thereof; CDR-H2 having a sequence of SEQ ID NO: 80 or a variant thereof; CDR-H3 having a sequence of SEQ ID NO: 73 or a variant thereof; and/or, a light chain variable region (VL) comprising 3 CDRs as follows: CDR-L1 having a sequence of SEQ ID NO: 74 or a variant thereof; CDR-L2 having a sequence of SEQ ID NO: 75 or a variant thereof; CDR-L3 having a sequence of SEQ ID NO: 76 or a variant thereof;
(3b) a heavy chain variable region (VH) comprising 3 CDRs as follows: CDR-H1 having a sequence of SEQ ID NO: 19 or a variant thereof; CDR-H2 having a sequence of SEQ ID NO: 20 or a variant thereof; CDR-H3 having a sequence of SEQ ID NO: 13 or a variant thereof; and/or, a light chain variable region (VL) comprising 3 CDRs as follows: CDR-L1 having a sequence of SEQ ID NO: 14 or a variant thereof; CDR-L2 having a sequence of SEQ ID NO: 15 or a variant thereof; CDR-L3 having a sequence of SEQ ID NO: 16 or a variant thereof;
(3c-i) a heavy chain variable region (VH) comprising 3 CDRs as follows: CDR-H1 having a sequence of SEQ ID NO: 33 or a variant thereof; CDR-H2 having a sequence of SEQ ID NO: 34 or a variant thereof; CDR-H3 having a sequence of SEQ ID NO: 27 or a variant thereof; and/or, a light chain variable region (VL) comprising 3 CDRs as follows: CDR-L1 having a sequence of SEQ ID NO: 28 or a variant thereof; CDR-L2 having a sequence of SEQ ID NO: 29 or a variant thereof; CDR-L3 having a sequence of SEQ ID NO: 30 or a variant thereof;
(3c-ii) a heavy chain variable region (VH) comprising 3 CDRs as follows: CDR-H1 having a sequence of SEQ ID NO: 101 or a variant thereof; CDR-H2 having a sequence of SEQ ID NO: 102 or a variant thereof; CDR-H3 having a sequence of SEQ ID NO: 27 or a variant thereof; and/or, a light chain variable region (VL) comprising 3 CDRs as follows: CDR-L1 having a sequence of SEQ ID NO: 97 or a variant thereof; CDR-L2 having a sequence of SEQ ID NO: 98 or a variant thereof; CDR-L3 having a sequence of SEQ ID NO: 30 or a variant thereof; or
(3d) a heavy chain variable region (VH) comprising 3 CDRs as follows: CDR-H1 having a sequence of SEQ ID NO: 62 or a variant thereof; CDR-H2 having a sequence of SEQ ID NO: 63 or a variant thereof; CDR-H3 having a sequence of SEQ ID NO: 64 or a variant thereof; and/or, a light chain variable region (VL) comprising 3 CDRs as follows: CDR-L1 having a sequence of SEQ ID NO: 59 or a variant thereof; CDR-L2 having a sequence of SEQ ID NO: 60 or a variant thereof; CDR-L3 having a sequence of SEQ ID NO: 61 or a variant thereof;
wherein the variant in any one of (3a), (3b), (3c-i), (3c-ii) and (3d) has a substitution, deletion or addition of one or several amino acids (e.g., a substitution, deletion or addition of 1, 2 or 3 amino acids) compared to the sequence from which the variant is derived. In certain embodiments, the substitution is a conservative substitution.

In certain embodiments, the antibody or the antigen-binding fragment thereof of the present disclosure comprises a heavy chain variable region (VH) and/or a light chain variable region (VL), wherein CDRs are defined by the AbM numbering system:
(4a) a heavy chain variable region (VH) comprising 3 CDRs as follows: CDR-H1 having a sequence of SEQ ID NO: 77 or a variant thereof; CDR-H2 having a sequence of SEQ ID NO: 78 or a variant thereof; CDR-H3 having a sequence of SEQ ID NO: 73 or a variant thereof; and/or, a light chain variable region (VL) comprising 3 CDRs as follows: CDR-L1 having a sequence of SEQ ID NO: 74 or a variant thereof; CDR-L2 having a sequence of SEQ ID NO: 75 or a variant thereof; CDR-L3 having a sequence of SEQ ID NO: 76 or a variant thereof;
(4b) a heavy chain variable region (VH) comprising 3 CDRs as follows: CDR-H1 having a sequence of SEQ ID NO: 17 or a variant thereof; CDR-H2 having a sequence of SEQ ID NO: 18 or a variant thereof; CDR-H3 having a sequence of SEQ ID NO: 13 or a variant thereof; and/or, a light chain variable region (VL) comprising 3 CDRs as follows: CDR-L1 having a sequence of SEQ ID NO: 14 or a variant thereof; CDR-L2 having a sequence of SEQ ID NO: 15 or a variant thereof; CDR-L3 having a sequence of SEQ ID NO: 16 or a variant thereof;
(4c-i) a heavy chain variable region (VH) comprising 3 CDRs as follows: CDR-H1 having a sequence of SEQ ID NO: 31 or a variant thereof; CDR-H2 having a sequence of SEQ ID NO: 32 or a variant thereof; CDR-H3 having a sequence of SEQ ID NO: 27 or a variant thereof; and/or, a light chain variable region (VL) comprising 3 CDRs as follows: CDR-L1 having a sequence of SEQ ID NO: 28 or a variant thereof; CDR-L2 having a sequence of SEQ ID NO: 29 or a variant thereof; CDR-L3 having a sequence of SEQ ID NO: 30 or a variant thereof;
(4c-ii) a heavy chain variable region (VH) comprising 3 CDRs as follows: CDR-H1 having a sequence of SEQ ID NO: 99 or a variant thereof; CDR-H2 having a sequence of SEQ ID NO: 100 or a variant thereof; CDR-H3 having a sequence of SEQ ID NO: 27 or a variant thereof; and/or, a light chain variable region (VL) comprising 3 CDRs as follows: CDR-L1 having a sequence of SEQ ID NO: 97 or a variant thereof; CDR-L2 having a sequence of SEQ ID NO: 98 or a variant thereof; CDR-L3 having a sequence of SEQ ID NO: 30 or a variant thereof; or
(4d) a heavy chain variable region (VH) comprising 3 CDRs as follows: CDR-H1 having a sequence of SEQ ID NO: 62 or a variant thereof; CDR-H2 having a sequence of SEQ ID NO: 63 or a variant thereof; CDR-H3 having a sequence of SEQ ID NO: 64 or a variant thereof; and/or, a light chain variable region (VL) comprising 3 CDRs as follows: CDR-L1 having a sequence of SEQ ID NO: 59 or a variant thereof; CDR-L2 having a sequence of SEQ ID NO: 60 or a variant thereof; CDR-L3 having a sequence of SEQ ID NO: 61 or a variant thereof;
wherein the variant in any one of (4a), (4b), (4c-i), (4c-ii) and (4d) has a substitution, deletion or addition of one or several amino acids (e.g., a substitution, deletion or addition of 1, 2 or 3 amino acids) compared to the sequence from which the variant is derived. In certain embodiments, the substitution is a conservative substitution.

In certain embodiments, the antibody or the antigen-binding fragment thereof of the present disclosure comprises:
(a-i) VH comprising a sequence as set forth in SEQ ID NO: 54 or a variant thereof and/or VL comprising a sequence as set forth in SEQ ID NO: 55 or a variant thereof;
(a-ii) VH comprising a sequence as set forth in SEQ ID NO: 52 or a variant thereof and/or VL comprising a sequence as set forth in SEQ ID NO: 53 or a variant thereof;
(b-i) VH comprising a sequence as set forth in SEQ ID NO: 3 or a variant thereof and/or VL comprising a sequence as set forth in SEQ ID NO: 4 or a variant thereof;
(b-ii) VH comprising a sequence as set forth in SEQ ID NO: 1 or a variant thereof and/or VL comprising a sequence as set forth in SEQ ID NO: 2 or a variant thereof;
(c-i) VH comprising a sequence as set forth in SEQ ID NO: 9 or a variant thereof and/or VL comprising a sequence as set forth in SEQ ID NO: 10 or a variant thereof;
(c-ii) VH comprising a sequence as set forth in SEQ ID NO: 7 or a variant thereof and/or VL comprising a sequence as set forth in SEQ ID NO: 8 or a variant thereof;
(d-i) VH comprising a sequence as set forth in SEQ ID NO: 50 or a variant thereof and/or VL comprising a sequence as set forth in SEQ ID NO: 51 or a variant thereof; or
(d-ii) VH comprising a sequence as set forth in SEQ ID NO: 48 or a variant thereof and/or VL comprising a sequence as set forth in SEQ ID NO: 49 or a variant thereof;
wherein the variant has at least 80%, at least 85%, at least 90%, at least 91%, at least 92%, at least 93%, at least 94%, at least 95%, at least 96%, at least 97%, at least 98%, at least 99% or 100% sequence identity to the sequence from which the variant is derived, or has a substitution, deletion or addition of one or several amino acids (e.g., a substitution, deletion or addition of 1, 2, 3, 4 or 5 amino acids) compared to the sequence from which the variant is derived; preferably, the substitution is a conservative substitution.

In certain embodiments of the antibody or the antigen-binding fragment disclosed herein, the heavy chain constant domain may comprise a C-terminal lysine or lack a C-terminal lysine or a C-terminal glycine-lysine dipeptide. In some embodiments of the antibody or the antigen-binding fragment thereof, the N-terminal amino acid of the antibody or the antigen-binding fragment thereof may be cyclized to pyroglutamic acid. In some embodiments of the antibody or the antigen-binding fragment thereof, the N-terminal amino acid of the antibody or the antigen-binding fragment thereof may be cyclized to pyroglutamic acid.

As known to those skilled in the art pyroglutamic acid is the conjugate acid of pyroglutamate and is in equilibrium with pyroglutamate in solution.

In certain embodiments, provided herein is a composition comprising the antibodies or the antigen-binding fragments disclosed herein, wherein the antibodies or the antigen-binding fragments may independently comprise a C-terminal lysine, lack a C-terminal lysine, lack a C-terminal glycine-lysine, and/or comprise an N-terminal glutamine or glutamic acid, an N-terminal amino acid cyclized to pyroglutamic acid or an N-terminal amino acid cyclized to pyroglutamate.

In certain embodiments, the antibodies or the antigen-binding fragments disclosed herein include antibodies or antigen-binding fragments that specifically bind to an antigen, and may include post-translational modifications thereof (e.g., cleavage of the C-terminal lysine in the heavy chain, and conversion of the N-terminal glutamine or glutamic acid in the heavy chain or light chain to pyroglutamic acid or pyroglutamate), which can occur during recombinant expression in a host cell (e.g., CHO cell) or during purification/storage.

In certain embodiments, the N-terminal glutamine of the VH comprising a sequence as set forth in SEQ ID NO: 54, 52, 3, 1, 9, 7, 50 or 48 or a variant thereof undergoes cyclization to form pyroglutamic acid or pyroglutamate. In certain embodiments, the N-terminal glutamine of the VH comprising a sequence as set forth in SEQ ID NO: 54, 3, 9 or 50 or a variant thereof undergoes cyclization to form pyroglutamic acid or pyroglutamate.

In certain embodiments, the antibody or the antigen-binding fragment thereof having the characteristic of any one of (1a), (2a), (3a), (4a), (a-i) or (a-ii) in the above embodiments further has a characteristic selected from:
(1) binding to ADAM9 (e.g., human or monkey ADAM9) with an EC₅₀ of less than about 100 ng/mL, e.g., less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less, wherein preferably, the EC₅₀ is measured by ELISA;
(2) binding to ADAM9 (e.g., human or monkey ADAM9) with a KD of less than about 100 nM, e.g., less than about 90 nM, 80 nM, 70 nM, 60 nM, 50 nM, 40 nM, 30 nM, 20 nM, 15 nM, 10 nM, 5 nM or less, wherein preferably, the KD is measured by bio-layer interferometry (BLI) (such as ForteBio Octet^{®});
(3) having CDC activity, e.g., inducing killing of a cell expressing ADAM9 (e.g., a tumor cell) by CDC;
(4) having no ADCC activity;
(5) inducing ADAM9 internalization, e.g., as determined by flow cytometry;
(6) having no binding to ADAM12 and ADAM15 proteins;
(7) inhibiting the proliferation of a cell (e.g., a tumor cell); and/or
(8) inhibiting tumor growth.

In certain embodiments, the antibody or the antigen-binding fragment thereof having the characteristic of any one of (1b-i), (1b-ii), (2b), (3b), (4b), (b-i) or (b-ii) in the above embodiments further has a characteristic selected from:
(1) binding to ADAM9 (e.g., human or monkey ADAM9) with an EC₅₀ of less than about 100 ng/mL, e.g., less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less, wherein preferably, the EC₅₀ is measured by ELISA;
(2) binding to ADAM9 (e.g., human or monkey ADAM9) with a KD of less than about 100 nM, e.g., less than about 90 nM, 80 nM, 70 nM, 60 nM, 50 nM, 40 nM, 30 nM, 20 nM, 15 nM, 10 nM, 5 nM or less, wherein preferably, the KD is measured by bio-layer interferometry (BLI) (such as ForteBio Octet^{®});
(3) having CDC activity, e.g., inducing killing of a cell expressing ADAM9 (e.g., a tumor cell) by CDC;
(4) having no ADCC activity;
(5) inducing ADAM9 internalization, e.g., as determined by flow cytometry;
(6) having no binding to ADAM12 and ADAM15 proteins;
(7) inhibiting the proliferation of a cell (e.g., a tumor cell); and/or
(8) inhibiting tumor growth.

In certain embodiments, the antibody or the antigen-binding fragment thereof having the characteristic of any one of (1c-i), (1c-ii), (2c-i), (2c-ii), (3c-i), (3c-ii), (4c-i), (4c-ii), (c-i) or (c-ii) in the above embodiments further has a characteristic selected from:
(1) binding to ADAM9 (e.g., human or monkey ADAM9) with an EC₅₀ of less than about 100 ng/mL, e.g., less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less, wherein preferably, the EC₅₀ is measured by ELISA;
(2) binding to ADAM9 (e.g., human or monkey ADAM9) with a KD of less than about 100 nM, e.g., less than about 90 nM, 80 nM, 70 nM, 60 nM, 50 nM, 40 nM, 30 nM, 20 nM, 15 nM, 10 nM, 5 nM or less, wherein preferably, the KD is measured by bio-layer interferometry (BLI) (such as ForteBio Octet^{®});
(3) having CDC activity, e.g., inducing killing of a cell expressing ADAM9 (e.g., a tumor cell) by CDC;
(4) having no ADCC activity;
(5) inducing ADAM9 internalization, e.g., as determined by flow cytometry;
(6) having no binding to ADAM12 and ADAM15 proteins;
(7) inhibiting the proliferation of a cell (e.g., a tumor cell); and/or
(8) inhibiting tumor growth.

In certain embodiments, the antibody or the antigen-binding fragment thereof having the characteristic of any one of (1d), (2d), (3b), (3d), (4d), (d-i) or (d-ii) in the above embodiments further has a characteristic selected from:
(1) binding to ADAM9 (e.g., human or monkey ADAM9) with an EC₅₀ of less than about 100 ng/mL, e.g., less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less, wherein preferably, the EC₅₀ is measured by ELISA;
(2) binding to ADAM9 (e.g., human or monkey ADAM9) with a KD of less than about 100 nM, e.g., less than about 90 nM, 80 nM, 70 nM, 60 nM, 50 nM, 40 nM, 30 nM, 20 nM, 15 nM, 10 nM, 5 nM or less, wherein preferably, the KD is measured by bio-layer interferometry (BLI) (such as ForteBio Octet^{®});
(3) having CDC activity, e.g., inducing killing of a cell expressing ADAM9 (e.g., a tumor cell) by CDC;
(4) having no ADCC activity;
(5) inducing ADAM9 internalization, e.g., as determined by flow cytometry;
(6) having no binding to ADAM12 and ADAM15 proteins;
(7) inhibiting the proliferation of a cell (e.g., a tumor cell); and/or
(8) inhibiting tumor growth.

In certain embodiments, the antibody or the antigen-binding fragment thereof according to any one of the above embodiments may comprise a constant region from or derived from a human immunoglobulin.

In certain embodiments, the heavy chain of the antibody or the antigen-binding fragment thereof comprises a heavy chain constant region from or derived from a human immunoglobulin (e.g., IgG1, IgG2, IgG3 or IgG4). In certain embodiments, the heavy chain of the antibody or the antigen-binding fragment thereof comprises a wild-type Fc region, or a mutated or chemically modified Fc region that has an altered effector function (e.g., decreased ADCC activity) compared to the wild-type Fc region. In certain exemplary embodiments, the antibody or the antigen-binding fragment thereof of the present disclosure comprises a variant of the human IgG1 heavy chain constant region, wherein the variant has the following substitutions compared to the wild-type sequence from which the variant is derived: Leu234Ala, Leu235Ala and Gly237Ala (positions according to the EU numbering system). In such embodiments, the antibody or the antigen-binding fragment thereof of the present disclosure has reduced ADCC activity. In certain embodiments, the heavy chain of the antibody or the antigen-binding fragment thereof comprises a sequence as set forth in SEQ ID NO: 5 or a variant thereof, wherein the variant has a conservative substitution of up to 20 amino acids (e.g., a conservative substitution of up to 15, up to 10, or up to 5 amino acids; e.g., a conservative substitution of 1, 2, 3, 4 or 5 amino acids) compared thereto. In certain embodiments, the heavy chain constant region (CH) as set forth in SEQ ID NO: 5, or the variant thereof lacks a C-terminal lysine.

In certain embodiments, the light chain of the antibody or the antigen-binding fragment thereof comprises a light chain constant region from or derived from a human immunoglobulin (e.g., κ or λ). In certain embodiments, the light chain of the antibody or the antigen-binding fragment thereof comprises a sequence as set forth in SEQ ID NO: 6 or a variant thereof, wherein the variant has a conservative substitution of up to 20 amino acids (e.g., a conservative substitution of up to 15, up to 10, or up to 5 amino acids; e.g., a conservative substitution of 1, 2, 3, 4 or 5 amino acids) compared thereto.

In certain embodiments, the antibody or the antigen-binding fragment thereof of the present disclosure comprises:
(1) a heavy chain comprising VH set forth in SEQ ID NO: 54 and a heavy chain constant region (CH) set forth in SEQ ID NO: 5; and a light chain comprising VL set forth in SEQ ID NO: 55 and a light chain constant region (CL) set forth in SEQ ID NO: 6;
(2) a heavy chain comprising VH set forth in SEQ ID NO: 52 and a heavy chain constant region (CH) set forth in SEQ ID NO: 5; and a light chain comprising VL set forth in SEQ ID NO: 53 and a light chain constant region (CL) set forth in SEQ ID NO: 6;
(3) a heavy chain comprising VH set forth in SEQ ID NO: 3 and a heavy chain constant region (CH) set forth in SEQ ID NO: 5; and a light chain comprising VL set forth in SEQ ID NO: 4 and a light chain constant region (CL) set forth in SEQ ID NO: 6;
(4) a heavy chain comprising VH set forth in SEQ ID NO: 1 and a heavy chain constant region (CH) set forth in SEQ ID NO: 5; and a light chain comprising VL set forth in SEQ ID NO: 2 and a light chain constant region (CL) set forth in SEQ ID NO: 6;
(5) a heavy chain comprising VH set forth in SEQ ID NO: 9 and a heavy chain constant region (CH) set forth in SEQ ID NO: 5; and a light chain comprising VL set forth in SEQ ID NO: 10 and a light chain constant region (CL) set forth in SEQ ID NO: 6;
(6) a heavy chain comprising VH set forth in SEQ ID NO: 7 and a heavy chain constant region (CH) set forth in SEQ ID NO: 5; and a light chain comprising VL set forth in SEQ ID NO: 8 and a light chain constant region (CL) set forth in SEQ ID NO: 6;
(7) a heavy chain comprising VH set forth in SEQ ID NO: 50 and a heavy chain constant region (CH) set forth in SEQ ID NO: 5; and a light chain comprising VL set forth in SEQ ID NO: 51 and a light chain constant region (CL) set forth in SEQ ID NO: 6; or
(8) a heavy chain comprising VH set forth in SEQ ID NO: 48 and a heavy chain constant region (CH) set forth in SEQ ID NO: 5; and a light chain comprising VL set forth in SEQ ID NO: 49 and a light chain constant region (CL) set forth in SEQ ID NO: 6.

In certain embodiments, the antibody or the antigen-binding fragment thereof of the present disclosure comprises:
(1) a heavy chain having a sequence as set forth in SEQ ID NO: 88 and a light chain having a sequence as set forth in SEQ ID NO: 89;
(2) a heavy chain having a sequence as set forth in SEQ ID NO: 40 and a light chain having a sequence as set forth in SEQ ID NO: 41; or,
(3) a heavy chain having a sequence as set forth in SEQ ID NO: 42 and a light chain having a sequence as set forth in SEQ ID NO: 43; or,
(4) a heavy chain having a sequence as set forth in SEQ ID NO: 86 and a light chain having a sequence as set forth in SEQ ID NO: 87.

In certain embodiments, the N-terminal glutamine of the heavy chain having a sequence as set forth in SEQ ID NO: 88, 40, 42 or 86 or a variant thereof undergoes cyclization to form pyroglutamic acid or pyroglutamate.

In certain embodiments, the antibody or the antigen-binding fragment thereof according to any one of the above embodiments is a murine antibody, a chimeric antibody or a humanized antibody.

In certain embodiments, the variable region of the antibody or the antigen-binding fragment thereof according to any one of the above embodiments is of human origin.

In certain embodiments, the antibody or the antigen-binding fragment thereof according to any one of the above embodiments is selected from ScFv, Fab, Fab', F(ab')2, Fab'-SH, an Fv fragment, disulfide-linked Fv (dsFv), a diabody, a bispecific antibody and a multispecific antibody.

In certain embodiments, the antibody or the antigen-binding fragment thereof according to any one of the above embodiments carries a label. In certain embodiments, the antibody or the antigen-binding fragment thereof carries a detectable label, e.g., an enzyme (e.g., horseradish peroxidase), a radionuclide, a fluorescent dye, a luminescent substance (e.g., a chemiluminescent substance) or a biotin.

The present disclosure further provides the use of the antibody or the antigen-binding fragment provided herein or the pharmaceutical composition thereof for the treatment of tumors.

### Derived antibodies

The antibody or the antigen-binding fragment thereof of the present disclosure can be derivatized, e.g., linked to another molecule (e.g., another polypeptide or protein). In general, derivatization (e.g., labeling) of an antibody or an antigen-binding fragment thereof does not adversely affect the binding thereof to ADAM9, particularly human ADAM9. Thus, the antibody or the antigen-binding fragment thereof of the present disclosure is also intended to include such derivatized forms. For example, the antibody or the antigen-binding fragment thereof of the present disclosure can be functionally linked (by chemical conjugation, genetic fusion, non-covalent linkage or other means) to one or more other molecular groups, e.g., another antibody (e.g., to form a bispecific antibody), a detection reagent, a pharmaceutical reagent and/or a protein or polypeptide (e.g., avidin or polyhistidine tag) capable of mediating the binding of an antibody or an antigen-binding fragment to another molecule.

As one of the antibody derivatives, the present disclosure provides a conjugate comprising the antibody or the antigen-binding fragment thereof of the present disclosure, and a conjugate moiety.

In certain embodiments, the conjugate moiety is selected from a detectable label. The detectable label of the present disclosure may be any substance that can be detected by fluorescent, spectroscopic, photochemical, biochemical, immunological, electrical, optical, or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., 3H, 125I, 35S, 14C or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7 or Alexa 750)), acridinium ester compounds, magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels. In certain embodiments, such labels can be suitable for use in immunological detection (e.g., enzyme-linked immunoassay, radioimmunoassay, fluorescence immunoassay, chemiluminescence immunoassay, etc.). In certain embodiments, the detectable label is selected from a radioisotope, a fluorescent substance, a luminescent substance, a colored substance or an enzyme. In certain embodiments, the detectable label described above may be linked to the antibody or the antigen-binding fragment thereof of the present disclosure via linkers of different lengths to reduce potential steric hindrance.

In certain embodiments, the conjugate moiety is selected from a therapeutic agent. In certain embodiments, the therapeutic agent is preferably an anti-tumor agent, e.g., a cytotoxic agent, a cytokine, a toxin or a radionuclide.

In certain embodiments, the conjugate moiety is selected from a substance capable of improving the biological properties (e.g., increasing serum half-life) of the antibody. For example, the conjugate moiety may be a chemical group (e.g., polyethylene glycol (PEG), methyl or ethyl) or a glycosyl group.

As one of the antibody derivatives, the present disclosure provides a multispecific antibody comprising the antibody or the antigen-binding fragment thereof of the present disclosure.

In certain embodiments, the multispecific antibody comprises the antibody or the antigen-binding fragment thereof of the present disclosure as a first antigen-binding domain, and further comprises at least one second antigen-binding domain against other targets.

In certain embodiments, the antigen-binding domains of the multispecific antibody retain their own antigen-binding specificity.

In certain embodiments, the multispecific antibody is a bispecific antibody or a trispecific antibody or a tetraspecific antibody.

As one of the antibody derivatives, the present disclosure provides a chimeric antigen receptor comprising the antibody or the antigen-binding fragment thereof of the present disclosure. In certain embodiments, the chimeric antigen receptor comprises the antibody or the antigen-binding fragment thereof of the present disclosure (e.g., ScFv) as an extracellular antigen-binding domain that specifically binds to ADAM9, and a transmembrane domain and one or more intracellular T cell signaling domains. The present disclosure further provides a host cell (e.g., immune cell, such as T lymphocyte, NK cell, DC cell or macrophage) that contains or expresses the chimeric antigen receptor.

### Preparation of antibodies

The antibody of the present disclosure can be prepared by various methods known in the art, for example, obtained by genetic engineering recombinant technology. For example, DNA molecules encoding the heavy chain and light chain genes of the antibody of the present disclosure are obtained by chemical synthesis or PCR amplification. The obtained DNA molecules are inserted into an expression vector, which is then transfected into host cells. Then, the transfected host cells are cultured under specific conditions, and the antibody of the present disclosure is expressed.

The antigen-binding fragments of the present disclosure can be obtained by hydrolysis of intact antibody molecules (see Morimoto et al., J. Biochem. Biophys. Methods 24: 107-117 (1992) and Brennan et al., Science 229: 81 (1985)). In addition, these antigen-binding fragments can also be produced directly from recombinant host cells (reviewed in Hudson, Curr. Opin. Immunol. 11: 548-557 (1999); Little et al., Immunol. Today, 21: 364-370 (2000)). For example, Fab' fragments can be obtained directly from the host cells; and Fab' fragments can be chemically conjugated to form F(ab')₂fragments (Carter et al., Bio/Technology, 10: 163-167 (1992)). Moreover, Fv, Fab or F(ab')₂ fragments can also be obtained directly by direct isolation from recombinant host cell cultures. Other techniques for preparing these antigen-binding fragments are fully known to those of ordinary skill in the art.

Thus, in another aspect, the present disclosure provides an isolated nucleic acid molecule, which comprises a nucleotide sequence encoding the antibody or the antigen-binding fragment thereof of the present disclosure, or a heavy chain variable region and/or light chain variable region thereof. According to codon degeneracy in the art, in certain embodiments, the nucleotide sequence can be substituted according to codon degeneracy. In certain embodiments, the nucleotide sequence is codon-optimized.

In certain embodiments, the isolated nucleic acid molecule comprises a nucleic acid molecule encoding the heavy chain variable region of the antibody, and/or a nucleic acid molecule encoding the light chain variable region of the antibody, wherein the nucleic acid molecule encoding the heavy chain variable region of the antibody comprises: (i) a nucleotide sequence set forth in SEQ ID NO: 92, (ii) a sequence substantially identical to SEQ ID NO: 92 (e.g., a sequence having at least about 85%, 90%, 95%, 99% or higher sequence identity to SEQ ID NO: 92, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 93, (v) a sequence substantially identical to SEQ ID NO: 93 (e.g., a sequence having at least about 85%, 90%, 95%, 99% or higher sequence identity to SEQ ID NO: 93, or a sequence having one or more nucleotide substitutions), or (vi) a degenerate sequence of (iv) or (v) above.

In certain embodiments, the isolated nucleic acid molecule comprises a nucleic acid molecule encoding the heavy chain variable region of the antibody, and/or a nucleic acid molecule encoding the light chain variable region of the antibody, wherein the nucleic acid molecule encoding the heavy chain variable region of the antibody comprises: (i) a nucleotide sequence set forth in SEQ ID NO: 44, (ii) a sequence substantially identical to SEQ ID NO: 44 (e.g., a sequence having at least about 85%, 90%, 95%, 99% or higher sequence identity to SEQ ID NO: 44, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 45, (v) a sequence substantially identical to SEQ ID NO: 45 (e.g., a sequence having at least about 85%, 90%, 95%, 99% or higher sequence identity to SEQ ID NO: 45, or a sequence having one or more nucleotide substitutions), or (vi) a degenerate sequence of (iv) or (v) above.

In certain embodiments, the isolated nucleic acid molecule comprises a nucleic acid molecule encoding the heavy chain variable region of the antibody, and/or a nucleic acid molecule encoding the light chain variable region of the antibody, wherein the nucleic acid molecule encoding the heavy chain variable region of the antibody comprises: (i) a nucleotide sequence set forth in SEQ ID NO: 46, (ii) a sequence substantially identical to SEQ ID NO: 46 (e.g., a sequence having at least about 85%, 90%, 95%, 99% or higher sequence identity to SEQ ID NO: 46, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 47, (v) a sequence substantially identical to SEQ ID NO: 47 (e.g., a sequence having at least about 85%, 90%, 95%, 99% or higher sequence identity to SEQ ID NO: 47, or a sequence having one or more nucleotide substitutions), or (vi) a degenerate sequence of (iv) or (v) above.

In certain embodiments, the isolated nucleic acid molecule comprises a nucleic acid molecule encoding the heavy chain variable region of the antibody, and/or a nucleic acid molecule encoding the light chain variable region of the antibody, wherein the nucleic acid molecule encoding the heavy chain variable region of the antibody comprises: (i) a nucleotide sequence set forth in SEQ ID NO: 90, (ii) a sequence substantially identical to SEQ ID NO: 90 (e.g., a sequence having at least about 85%, 90%, 95%, 99% or higher sequence identity to SEQ ID NO: 90, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 91, (v) a sequence substantially identical to SEQ ID NO: 91 (e.g., a sequence having at least about 85%, 90%, 95%, 99% or higher sequence identity to SEQ ID NO: 91, or a sequence having one or more nucleotide substitutions), or (vi) a degenerate sequence of (iv) or (v) above.

In another aspect of the present disclosure, the present disclosure provides a vector (e.g., a cloning vector or an expression vector), which comprises the isolated nucleic acid molecule of the present disclosure. In certain embodiments, the vector of the present disclosure is, for example, a plasmid, a cosmid, a phage, a lentivirus, etc. In certain embodiments, the vector can express the antibody or the antigen-binding fragment thereof of the present disclosure in a subject (e.g., a mammal, e.g., a human).

In certain embodiments, the vector comprises a first nucleotide sequence encoding the heavy chain or heavy chain variable region of the antibody or the antigen-binding fragment thereof of the present disclosure and a second nucleotide sequence encoding the light chain or the light chain variable region thereof, wherein the first nucleotide sequence and the second nucleotide sequence are present on the same or different vectors. When the first nucleotide sequence and the second nucleotide sequence are present on different vectors, the vector of the present disclosure comprises a first vector comprising the first nucleotide sequence and a second vector comprising the second nucleotide sequence.

In certain embodiments, the antibody or the antigen-binding fragment thereof of the present disclosure can be used to construct a chimeric antigen receptor (CAR), which comprises an extracellular antigen-binding domain (e.g., ScFv) that specifically binds to ADAM9, a transmembrane domain, and one or more intracellular T cell signaling domains. In such embodiments, the isolated nucleic acid molecule of the present disclosure may comprise a nucleotide sequence encoding a chimeric antigen receptor, wherein the nucleotide sequence encoding the chimeric antigen receptor further comprises a nucleotide sequence encoding the antibody or the antigen-binding fragment thereof (e.g., ScFv) of the present disclosure. In certain embodiments, the isolated nucleic acid molecule of the present disclosure encodes a chimeric antigen receptor comprising the antigen-binding fragment (e.g., ScFv) of the antibody of the present disclosure.

In certain embodiments, the antibody or the antigen-binding fragment thereof of the present disclosure can be used to construct a chimeric antigen receptor-modified immune cell which comprises a chimeric antigen receptor (CAR) and an immune cell (e.g., T lymphocyte, NK cell, DC cell or macrophage).

In another aspect of the present disclosure, the present disclosure provides a host cell comprising the isolated nucleic acid molecule of the present disclosure or the vector of the present disclosure. The host cell may be a eukaryotic cell (e.g., mammalian cell, insect cell or yeast cell) or a prokaryotic cell (e.g., *Escherichia coli*). Suitable eukaryotic cells include, but are not limited to, NS0 cells, Vero cells, Hela cells, COS cells, CHO cells, ExpiCHO cells, HEK293 cells, Expi293 cells, BHK cells, and MDCKII cells. Suitable insect cells include, but are not limited to, Sf9 cells. In certain embodiments, the host cell of the present disclosure is a mammalian cell, e.g., CHO (e.g., CHO-K1, CHO-S, CHO DXB11, ExpiCHO, CHO DG44 or CHO-EBNA).

In certain embodiments, the host cell of the present disclosure may be a chimeric antigen receptor T cell (CAR-T). In such embodiments, the isolated nucleic acid molecule contained in the host cell may comprise a nucleotide sequence encoding a chimeric antigen receptor, wherein the nucleotide sequence encoding the chimeric antigen receptor further comprises a nucleotide sequence encoding the antibody or the antigen-binding fragment thereof (e.g., ScFv) of the present disclosure. In certain embodiments, the isolated nucleic acid molecule contained in the host cell encodes a chimeric antigen receptor comprising the antigen-binding fragment (e.g., ScFv) of the antibody of the present disclosure.

In another aspect of the present disclosure, provided is a method of preparing the antibody or the antigen-binding fragment thereof of the present disclosure, the method comprising culturing the host cell of the present disclosure under conditions that allow expression of the antibody or the antigen-binding fragment thereof, and recovering the antibody or the antigen-binding fragment thereof from a culture of the cultured host cell.

### Therapeutic uses

In another aspect of the present disclosure, provided is a pharmaceutical composition comprising the antibody or the antigen-binding fragment thereof, the nucleic acid molecule, the vector, the host cell, the conjugate, the multispecific antibody, or the chimeric antigen receptor or a host cell expressing the chimeric antigen receptor of the present disclosure, and a pharmaceutically acceptable carrier and/or excipient.

In certain embodiments, the pharmaceutical composition of the present disclosure comprises the antibody or the antigen-binding fragment thereof of the present disclosure, and a pharmaceutically acceptable carrier and/or excipient.

In certain embodiments, the pharmaceutical composition of the present disclosure comprises the vector or the host cell of the present disclosure, and a pharmaceutically acceptable carrier and/or excipient. In such embodiments, the isolated nucleic acid molecule contained in the vector comprises a nucleotide sequence encoding a chimeric antigen receptor, wherein the nucleotide sequence encoding the chimeric antigen receptor further comprises a nucleotide sequence encoding the antibody or the antigen-binding fragment thereof (e.g., ScFv) of the present disclosure; the host cell comprises the isolated nucleic acid molecule or the vector described above. In certain embodiments, the isolated nucleic acid molecule encodes a chimeric antigen receptor comprising the antigen-binding fragment (e.g., ScFv) of the antibody of the present disclosure. In certain embodiments, the host cell is an immune cell, e.g., a T cell. In certain embodiments, the host cell is a chimeric antigen receptor T cell (CAR-T).

In certain embodiments, the pharmaceutical composition may also comprise an additional pharmaceutically active agent. In certain embodiments, the additional pharmaceutically active agent is a drug having anti-tumor activity. In certain embodiments, the additional pharmaceutically active agent is selected from an ADAM9 inhibitor, an EGFR inhibitor, an HER2 inhibitor, an HER3 inhibitor, an HER4 inhibitor, an IGFR-1 inhibitor, an mTOR inhibitor, a PI3 kinase inhibitor, a c-met inhibitor or a VEGF inhibitor, a chemotherapeutic agent or any combination thereof. In certain embodiments, the antibody or the antigen-binding fragment thereof of the present disclosure and the additional pharmaceutically active agent are provided as separate components or as a mixed component. Thus, the antibody or the antigen-binding fragment thereof of the present disclosure and the additional pharmaceutically active agent may be administered simultaneously, separately or sequentially.

In certain embodiments, the antibody or the antigen-binding fragment thereof, the nucleic acid molecule, the vector, the host cell, the conjugate, the multispecific antibody, or the chimeric antigen receptor or a host cell expressing the chimeric antigen receptor in the pharmaceutical composition of the present disclosure is sufficient (e.g., in a subject) to:
(a) inhibit the proliferation of a cell (e.g., a tumor cell);
(b) inhibit tumor growth;
(c) induce and/or increase antibody-dependent cytotoxic activity;
(d) inhibit ADAM9-mediated signaling;
(e) prevent and/or treat an ADAM9-mediated disease/disorder; or
(f) any combination of (a)-(e).

In certain embodiments, the ADAM9-mediated disease/disorder is a tumor, e.g., a tumor expressing ADAM9. In certain embodiments, the tumor is selected from glioma, cervical cancer, oral squamous cell carcinoma, liver cancer, melanoma, prostate cancer, pancreatic ductal adenocarcinoma, gastric cancer, lung cancer such as non-small cell lung cancer, pancreatic cancer, ovarian cancer, colorectal cancer, head and neck cancer, breast cancer such as triple-negative breast cancer, or any combination thereof.

In another aspect of the present disclosure, provided is the use of the antibody or the antigen-binding fragment thereof, the nucleic acid molecule, the vector, the host cell, the conjugate, the multispecific antibody, the chimeric antigen receptor or a host cell expressing the chimeric antigen receptor, or the pharmaceutical composition of the present disclosure in the preparation of a drug for: inhibiting cell proliferation, or preventing and/or treating and/or adjuvantly treating a tumor, a neurodegenerative disease, a retinal disease or inflammation.

In certain embodiments, the drug is used for inhibiting the proliferation of a cell expressing ADAM9 (e.g., a tumor cell).

In another aspect of the present disclosure, provided is a method for inhibiting the proliferation of a cell, the method comprising contacting the cell with the antibody or the antigen-binding fragment thereof, the nucleic acid molecule, the vector, the host cell, the conjugate, the multispecific antibody, the chimeric antigen receptor or a host cell expressing the chimeric antigen receptor, or the pharmaceutical composition of the present disclosure. In certain embodiments, the cell is a cell expressing ADAM9, e.g., a tumor cell.

In another aspect of the present disclosure, provided is a method for preventing and/or treating and/or adjuvantly treating a tumor, a neurodegenerative disease, a retinal disease or inflammation in a subject, the method comprising administering to a subject in need thereof an effective amount of the antibody or the antigen-binding fragment thereof, the nucleic acid molecule, the vector, the host cell, the conjugate, the multispecific antibody, the chimeric antigen receptor or a host cell expressing the chimeric antigen receptor, or the pharmaceutical composition of the present disclosure.

In certain embodiments, the method further comprises administering to the subject a second therapy selected from surgery, chemotherapy, radiotherapy, immunotherapy, gene therapy, DNA therapy, RNA therapy, nanotherapy, viral therapy, adjuvant therapy and any combination thereof. In certain embodiments, the second therapy may be applied concurrently with, separately from, or sequentially to the above method.

In any one of the above embodiments, the tumor involved in the antibody or the antigen-binding fragment thereof, the nucleic acid molecule, the vector, the host cell, the conjugate, the multispecific antibody, the chimeric antigen receptor or a host cell expressing the chimeric antigen receptor, or the pharmaceutical composition of the present disclosure may be of any tumor type. In certain embodiments, the tumor involved in the antibody or the antigen-binding fragment thereof, the nucleic acid molecule, the vector, the host cell, the conjugate, the multispecific antibody, the chimeric antigen receptor or a host cell expressing the chimeric antigen receptor, or the pharmaceutical composition of the present disclosure is an ADAM9-positive tumor. In certain embodiments, the tumor involved in the antibody or the antigen-binding fragment thereof, the nucleic acid molecule, the vector, the host cell, the conjugate, the multispecific antibody, the chimeric antigen receptor or a host cell expressing the chimeric antigen receptor, or the pharmaceutical composition of the present disclosure is selected from glioma, cervical cancer, oral squamous cell carcinoma, liver cancer, melanoma, prostate cancer, pancreatic ductal adenocarcinoma, gastric cancer, lung cancer such as non-small cell lung cancer, pancreatic cancer, ovarian cancer, colorectal cancer, head and neck cancer, breast cancer such as triple-negative breast cancer, or any combination thereof.

The antibody or the antigen-binding fragment thereof of the present disclosure or the pharmaceutical composition of the present disclosure may be formulated into any dosage form known in the medical field, e.g., a tablet, a pill, a suspension, an emulsion, a solution, a gel, a capsule, a powder, a granule, an elixir, a troche, a suppository, an injection (including an injection solution, a sterile powder for injection and a concentrated solution for injection), an inhalant, a spray, etc. The preferred dosage form depends on the intended mode of administration and therapeutic use. The pharmaceutical composition of the present disclosure should be sterile and stable under the conditions of manufacture and storage. One preferred dosage form is an injection. Such injections may be sterile injection solutions. For example, sterile injection solutions can be prepared by the method described below: a necessary dosage of the antibody of the present disclosure is incorporated in an appropriate solvent, and optionally, other desired ingredients (including, but not limited to, pH regulators, surfactants, adjuvants, ionic strength enhancers, isotonic agents, preservatives, diluents or any combination thereof) are added at the same time, followed by sterilization by filtration. In addition, sterile injection solutions can be prepared as sterile lyophilized powders (e.g., by vacuum drying or lyophilizing) for ease of storage and use. Such sterile lyophilized powders can be dispersed in a suitable carrier, e.g., sterile pyrogen-free water, prior to use.

In addition, the antibody or the antigen-binding fragment thereof of the present disclosure may be present in a pharmaceutical composition in unit dosage form for ease of administration.

The antibody or the antigen-binding fragment thereof or the pharmaceutical composition of the present disclosure may be administered by any suitable method known in the art, including, but not limited to, oral, buccal, sublingual, ocular, topical, parenteral, rectal, intrathecal, intracisternal, inguinal, intravesical, topical (e.g., powder, ointment, or drop), or nasal route. However, for many therapeutic uses, the preferred route/mode of administration is parenteral administration (e.g., intravenous injection, subcutaneous injection, intraperitoneal injection or intramuscular injection). It should be understood by the skilled person that the route and/or mode of administration will vary depending on the intended purpose. In a preferred embodiment, the antibody or the antigen-binding fragment thereof or the pharmaceutical composition of the present disclosure is administered by intravenous infusion or injection.

The pharmaceutical composition of the present disclosure may comprise a "therapeutically effective amount" or a "prophylactically effective amount" of the antibody or the antigen-binding fragment thereof of the present disclosure. A "prophylactically effective amount" refers to an amount sufficient to prevent, stop or delay the onset of a disease. A "therapeutically effective amount" refers to an amount sufficient to cure or at least partially prevent a disease and complications thereof in a patient suffering from the disease. The therapeutically effective amount of the antibody or the antigen-binding fragment thereof of the present disclosure may vary depending on the following factors: the severity of the disease to be treated, the overall status of the patient's own immune system, the general situation of the patient such as age, body weight and gender, the mode of administration of the drug, other concurrent treatments, etc.

In the present disclosure, the administration regimen may be adjusted to achieve the optimal desired response (e.g., a therapeutic or prophylactic response). For example, a single dose may be administered, multiple doses may be administered over a period of time, or the dose may be proportionally reduced or increased depending on the urgency of the treatment situation.

In the present disclosure, the subject may be a mammal, e.g., a human.

### Detection applications

The antibody or the antigen-binding fragment thereof of the present disclosure can specifically bind to ADAM9, and thus can be used to detect the presence or level of ADAM9 in a sample.

Thus, in another aspect, the present disclosure provides a kit comprising the antibody or the antigen-binding fragment thereof of the present disclosure. In certain embodiments, the antibody or the antigen-binding fragment thereof of the present disclosure carries a detectable label. In a preferred embodiment, the kit further comprises a second antibody that specifically recognizes the antibody or the antigen-binding fragment thereof of the present disclosure. Preferably, the second antibody further comprises a detectable label.

In the present disclosure, the detectable label may be any substance that can be detected by fluorescent, spectroscopic, photochemical, biochemical, immunological, electrical, optical, or chemical means. It is particularly preferred that such labels can be suitable for use in immunological detection (e.g., enzyme-linked immunoassay, radioimmunoassay, fluorescence immunoassay, chemiluminescence immunoassay, etc.). Such labels are well known in the art, and include, but are not limited to, enzymes (e.g., horseradish peroxidase, alkaline phosphatase, β-galactosidase, urease, glucose oxidase, etc.), radionuclides (e.g., 3H, 125I, 35S, 14C or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7 or Alexa 750)), acridinium ester compounds, magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels. In certain embodiments, the detectable label described above may be linked to the antibody of the present disclosure via linkers of different lengths to reduce potential steric hindrance.

In another aspect, the present disclosure provides a method for detecting the presence or level of ADAM9 in a sample, the method comprising a step of using the antibody or the antigen-binding fragment thereof of the present disclosure. In a preferred embodiment, the antibody or the antigen-binding fragment thereof of the present disclosure further carries a detectable label. In another preferred embodiment, the method further comprises detecting the antibody or the antigen-binding fragment thereof of the present disclosure using a reagent carrying a detectable label. The method may be used for diagnostic purposes, or for non-diagnostic purposes (for example, the sample is a cell sample rather than a sample derived from a patient).

In certain embodiments, the method comprises contacting the sample with the antibody or the antigen-binding fragment thereof of the present disclosure under conditions that allow formation of a complex between the antibody or the antigen-binding fragment thereof and ADAM9, and detecting the formation of the complex.

Given the low expression or no expression of ADAM9 in normal tissues and its expression or high expression in some cancers, tumors can be diagnosed by detecting the presence or level of ADAM9 in a sample. Thus, in certain embodiments, the method is used for diagnosing a tumor, e.g., an ADAM9-positive tumor, e.g., glioma, cervical cancer, oral squamous cell carcinoma, liver cancer, melanoma, prostate cancer, pancreatic ductal adenocarcinoma, gastric cancer, lung cancer such as non-small cell lung cancer, pancreatic cancer, ovarian cancer, colorectal cancer, head and neck cancer, breast cancer such as triple-negative breast cancer, or any combination thereof.

In certain embodiments, the method comprises detecting the expression level of ADAM9 in a test sample from a subject and comparing the expression level with a reference value (e.g., a healthy control), wherein an increase in the expression level compared to the reference value is an indication of a tumor.

In another aspect, provided is the use of the antibody or the antigen-binding fragment thereof of the present disclosure in the preparation of a kit for detecting the presence or level of ADAM9 in a sample and/or diagnosing a tumor.

In another aspect, the present disclosure provides a diagnostic or therapeutic kit which comprises the antibody or the antigen-binding fragment thereof, the nucleic acid molecule, the vector, the host cell, the conjugate or the multispecific antibody of the present disclosure, and instructions for use. The kit may also contain an administration device for topical administration. The administration device includes a drug-loaded syringe or a needle-free device.

### Beneficial Effects

The antibodies of the present disclosure exhibit high binding affinity and extremely strong specificity for ADAM9, and have no ADCC activity, thereby effectively avoiding side effects caused by the ADCC function. The antibodies of the present disclosure can inhibit tumor cell proliferation, and therefore, the antibodies of the present disclosure have the potential for preventing and/or treating a tumor. In addition, the antibodies of the present disclosure are humanized antibodies that can be safely administered to a human subject without eliciting an immunogenic response. Therefore, the antibodies of the present disclosure have significant clinical value.

### Abbreviations

- CDR: Complementarity determining region in an immunoglobulin variable region
- FR: Antibody framework region: amino acid residues other than CDR residues in an antibody variable region
- VH: Antibody heavy chain variable region
- VL: Antibody light chain variable region
- IgG: Immunoglobulin G
- IMGT: A numbering system based on the international ImMunoGeneTics information system^{®} (IMGT) initiated by Lefranc et al., reference can be made to Lefranc et al., Dev. Comparat. Immunol. 27: 55-77, 2003.
- Kabat: An immunoglobulin alignment and numbering system proposed by Elvin A. Kabat (See, e.g., Kabat et al., Sequences of Proteins of Immunological Interest, 5th Ed. Public Health Service, National Institutes of Health, Bethesda, Md., 1991).
- Chothia: An immunoglobulin numbering system proposed by Chothia *et al.*, which is a classical rule for identifying CDR region boundaries based on the positions of structural loop regions (see, e.g., Chothia & Lesk (1987) J. Mol. Biol. 196: 901-917; Chothia et al. (1989) Nature 342: 878-883).
- AbM: A method for defining CDRs, which is derived from related research by Martin (Martin ACR, Cheetham JC, Rees AR (1989) Modelling antibody hypervariable loops: A combined algorithm. Proc Natl Acad Sci USA 86: 9268-9272).
- mAb: Monoclonal antibody
- EC₅₀: Concentration producing 50% efficacy or binding
- IC₅₀: Concentration producing 50% inhibition
- ELISA: Enzyme-linked immunosorbent assay
- PCR: Polymerase chain reaction
- HRP: Horseradish peroxidase
- K_{D}: Equilibrium dissociation constant
- Ka: Association rate constant
- Kd: Dissociation rate constant
- ADCC: Antibody-dependent cell-mediated cytotoxicity
- CDC: Complement-dependent cytotoxicity
- FACS: Fluorescence-activated cell sorting
- CDR-H1: Complementarity determining region 1 in an immunoglobulin heavy chain variable region
- CDR-H2: Complementarity determining region 2 in an immunoglobulin heavy chain variable region
- CDR-H3: Complementarity determining region 3 in an immunoglobulin heavy chain variable region
- CDR-L1: Complementarity determining region 1 in an immunoglobulin light chain variable region
- CDR-L2: Complementarity determining region 2 in an immunoglobulin light chain variable region
- CDR-L3: Complementarity determining region 3 in an immunoglobulin light chain variable region

### Definitions

In the present disclosure, the scientific and technical terms used herein have the meanings as commonly understood by those skilled in the art unless otherwise specified. Moreover, laboratory operation steps of cell culture, biochemistry, nucleic acid chemistry, immunology, etc., used herein are all conventional steps widely used in the corresponding art. Moreover, for better understanding of the present disclosure, definitions and explanations of related terms are provided below.

As used herein, the term "antibody" is used in the broadest sense and includes various antibody structures, including but not limited to monoclonal antibodies, polyclonal antibodies, multispecific antibodies (e.g., bispecific antibodies) and antibody fragments, as long as they exhibit the desired antigen-binding activity. For example, the "antibody" may be an immunoglobulin molecule consisting of two pairs of polypeptide chains, with each pair having a light chain (LC) and a heavy chain (HC). Antibody light chains can be classified as κ (kappa) and λ (lambda) light chains. Heavy chains can be classified as µ, δ, γ, α or ε, and the isotypes of an antibody are defined as IgM, IgD, IgG, IgA and IgE, respectively. Within light and heavy chains, the variable and constant regions are joined by a "J" region of about 12 or more amino acids, with the heavy chain further comprising a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of an antibody to an antigen, but exhibits a variety of effector functions. For example, the constant domain can mediate the binding of immunoglobulins to host tissues or factors, including various cells (e.g., effector cells) of the immune system and the first component of the classical complement system (C1q). VH and VL regions can also be subdivided into regions with high variability (called complementarity determining regions (CDRs)), which are interspersed with more conserved regions called framework regions (FRs). Each VH and VL consists of three CDRs and four FRs, arranged from the amino terminus to the carboxy terminus in the following order: FR1, CDR1, FR2, CDR2, FR3, CDR3 and FR4. The variable regions (VH and VL) of each heavy/light chain pair form an antigen-binding site. The distribution of amino acids in various regions or domains follows the definitions in: Kabat, Sequences of Proteins of Immunological Interest (National Institutes of Health, Bethesda, Md. (1987 and 1991)), or Chothia & Lesk (1987) J. Mol. Biol. 196: 901-917; and Chothia et al. (1989) Nature 342: 878-883.

Unless otherwise clearly defined in the context, the term "antibody" when referred to herein comprises not only an intact antibody but also an antigen-binding fragment of an antibody.

The term "antibody" also includes embodiments in which the constant region of the heavy chain contains a C-terminal lysine, or lacks a C-terminal lysine or a C-terminal glycine-lysine dipeptide. The term also includes embodiments in which the N-terminal amino acid of the variable region of an antibody has been cyclized to pyroglutamate. Thus, in the composition comprising the antibodies disclosed herein, the antibodies may independently comprise a C-terminal lysine, lack a C-terminal lysine, lack a C-terminal glycine-lysine, and/or comprise an N-terminal glutamine or glutamic acid or an N-terminal amino acid cyclized to pyroglutamic acid.

As used herein, the term "complementarity determining region" or "CDR" refers to the amino acid residues in the variable regions of an antibody that are responsible for antigen binding. The exact boundaries of these amino acid residues can be defined according to various numbering systems known in the art, for example, according to the AbM numbering system (Martin ACR, Cheetham JC, Rees AR

(1989) Modelling antibody hypervariable loops: A combined algorithm. Proc Natl Acad Sci USA 86: 9268-9272) or the IMGT numbering system (Lefranc et al., Dev. Comparat. Immunol. 27: 55-77, 2003). For a given antibody, those skilled in the art will readily identify the CDRs defined by the numbering systems. In addition, the correspondence between different numbering systems is well known to those skilled in the art (see, e.g., Lefranc et al., Dev. Comparat. Immunol. 27: 55-77, 2003).

In the present disclosure, the CDRs contained in the antibody or the antigen-binding fragment thereof of the present disclosure can be determined according to various numbering systems known in the art. In certain embodiments, the CDRs contained in the antibody or the antigen-binding fragment thereof of the present disclosure are preferably determined by the IMGT, Kabat, Chothia or AbM numbering system.

The following general rules (published at www.bioinf.org.uk by the research group of Professor Andrew C.R. Martin) can be used to define CDRs in antibody sequences, including amino acids that specifically interact with amino acids constituting an antigenic epitope bound by an antibody. In rare cases, these generally constant features do not occur; however, the Cys residue is the most conserved feature.

| Rin g | Kabat | AbM | Chothia¹ | Chothia² | IMGT |
|---|---|---|---|---|---|
| L1 | L24--L34 | L24--L34 | L24--L34 | L30--L36 | L27--L32 |
| L2 | L50--L56 | L50--L56 | L50--L56 | L46--L55 | L50--L52 |
| L3 | L89--L97 | L89--L97 | L89--L97 | L89--L96 | L89--L97 |
| H1 | H31--H35B (Kabat numbering system)³ | H26-H35B | H26--H32..34 | H30--H35B | H26-H35B |
| H1 | H31--H35 (Chothia numbering system) | H26--H35 | H26--H32 | H30--H35 | H26--H33 |
| H2 | H50--H65 | H50--H58 | H52--H56 | H47--H58 | H51--H56 |
| H3 | H95--H102 | H95--H102 | H95--H102 | H93--H101 | H93-H102 |
| ¹ Some of these numbering systems (in particular the Chothia numbering system) differ depending on the public publications reviewed. | | | | | |
| ² Except for the Contact numbering system, which uses Chothia or Martin (extended Chothia) definitions, any other numbering system can be used for the definition of the above CDRs. | | | | | |
| ³ When numbering using the Kabat numbering system, the end of the Chothia CDR-H1 loop varies between H32 and H34, depending on the length of the loop. (This is because the Kabat numbering system positions the insertion sites at positions H35A and H35B). | | | | | |
| If both H35A and H35B are absent, the end of the loop is at H32; | | | | | |
| if only H35A is present, the end of the loop is at H33; | | | | | |
| if both H35A and H35B are present, the end of the loop is at H34. | | | | | |

The entire amino acid sequence of V_{H} is generally numbered according to Kabat, while the three CDRs within the variable region can be defined according to any one of the above numbering systems. In certain embodiments, amino acid positions in V_{H} can be numbered sequentially from amino acid position 1 to the end of the sequence, or according to Kabat. Unless otherwise stated, the amino acid positions in V_{H} and V_{L} described herein are defined according to sequential numbering.

The amino acid positions in the heavy chain constant region may be sequentially numbered from amino acid position 1 to the end of the sequence, or according to the Eu numbering system. The amino acid sequence of the IgG1 heavy chain constant region comprises 330 amino acids, sequentially numbered 1 to 330. The corresponding sequence numbered according to the Eu numbering system starts at position 118 and ends at position 447. Unless otherwise stated, the amino acid positions of the heavy and light chains described herein are defined according to sequential numbering.

As used herein, the term "framework region" or "FR" residues refer to those amino acid residues in the antibody variable region other than the CDR residues as defined above.

The term "antibody" is not limited by any particular method for producing an antibody. For example, the antibody includes a recombinant antibody, a monoclonal antibody and a polyclonal antibody. The antibody may be antibodies of different isotypes, e.g., an IgG (e.g., IgG1, IgG2, IgG3 or IgG4 subtype), IgA1, IgA2, IgD, IgE or IgM antibody.

As used herein, the term "antigen-binding fragment" of an antibody refers to a molecule other than an intact antibody that includes a portion of an intact antibody that binds to an antigen to which the intact antibody binds. For example, the antigen-binding fragment may be a polypeptide of a fragment of a full-length antibody that retains the ability to specifically bind to the same antigen to which the full-length antibody binds, and/or competes with the full-length antibody for specific binding to the antigen, which is also called "antigen-binding moiety". See generally, Fundamental Immunology, Ch. 7, Paul, W., ed., 2nd ed., Raven Press, N.Y. (1989), which is incorporated herein by reference in its entirety for all purposes. An antigen-binding fragment of an antibody can be generated by recombinant DNA techniques or by enzymatic or chemical cleavage of an intact antibody. Non-limiting examples of antigen-binding fragments include Fab, Fab', Fab'-SH, F(ab')₂, Fd, Fv, dAb and complementarity determining region (CDR) fragments, single-chain antibodies (e.g., scFv), chimeric antibodies, diabodies, linear antibodies, nanobodies (technology from Domantis), domain antibodies (technology from Ablynx), and polypeptides that comprise at least a portion of an antibody sufficient to confer specific antigen-binding ability on the polypeptide. Engineered antibody variants are reviewed in Holliger et al., 2005; Nat Biotechnol, 23: 1126-1136.

As used herein, the term "full-length antibody" refers to an antibody consisting of two "full-length heavy chains" or "heavy chains" and two "full-length light chains" or "light chains". The "full-length heavy chain" or "heavy chain" refers to a polypeptide chain that, in the direction from the N-terminus to the C-terminus, consists of a heavy chain variable region (VH), a heavy chain constant region CH1 domain, a hinge region (HR), a heavy chain constant region CH2 domain and a heavy chain constant region CH3 domain. Moreover, when the full-length antibody is of the IgE isotype, the heavy chain optionally further comprises a heavy chain constant region CH4 domain. Preferably, "full-length heavy chain" is a polypeptide chain consisting of VH, CH1, HR, CH2 and CH3 in the direction from the N-terminus to the C-terminus. "Full-length light chain" or "light chain" is a polypeptide chain consisting of a light chain variable region (VL) and a light chain constant region (CL) in the direction from the N-terminus to the C-terminus. Two pairs of full-length antibody chains are linked together via a disulfide bond between CL and CH1 and a disulfide bond between HRs of the two full-length heavy chains. The full-length antibody of the present disclosure may be from a single species, e.g., human. It may also be a chimeric antibody or a humanized antibody. The full-length antibody of the present disclosure comprises two antigen-binding sites formed by VH and VL pairs, respectively, and these two antigen-binding sites specifically recognize/bind to the same antigen.

As used herein, the term "Fab fragment" refers to an antibody fragment consisting of the VL, VH, CL and CH1 domains; the term "F(ab')2 fragment" refers to an antibody fragment comprising two Fab fragments linked via a disulfide bridge on the hinge region; the term "Fab' fragment" refers to a fragment obtained by reducing the disulfide bond linking two heavy chain fragments in the F(ab')2 fragment, which consists of an entire light chain and an Fd fragment of a heavy chain (consisting of VH and CH1 domains); and the term "Fab'-SH" refers to a Fab fragment containing a free sulfhydryl group.

As used herein, the term "Fv fragment" refers to an antibody fragment consisting of the VL and VH domains of a single arm of an antibody. The Fv fragment is generally considered as the minimum antibody fragment that can form a complete antigen-binding site. Generally, it is believed that the six CDRs confer an antibody with antigen-binding specificity. However, even a variable region (e.g., an Fd fragment, which contains only three CDRs specific for an antigen) is capable of recognizing and binding to an antigen, although its affinity may be lower than that of a complete binding site.

As used herein, the term "Fc fragment" refers to an antibody fragment formed by linking the second and third constant regions of a first heavy chain and the second and third constant regions of a second heavy chain of an antibody via a disulfide bond. The Fc fragment of an antibody has a variety of different functions, but is not involved in antigen binding.

As used herein, the term "scFv" refers to a single polypeptide chain comprising VL and VH domains, wherein the VL and VH are linked via a linker (see, e.g., Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Roseburg and Moore eds., Springer-Verlag, New York, pp. 269-315 (1994)). Such scFv molecules may have a general structure of NH2-VL-linker-VH-COOH or NH2-VH-linker-VL-COOH. Suitable linkers in the prior art consist of a repeated GGGGS amino acid sequence or a variant thereof. For example, a linker having an amino acid sequence (GGGGS)4 may be used, and a variant thereof may also be used (Holliger et al. (1993), Proc. Natl. Acad. Sci. USA 90: 6444-6448). Other linkers that may be used in the present disclosure are described by Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56; and Roovers et al. (2001), Cancer Immunol*.* In some cases, a disulfide bond may also exist between the VH and VL of the scFv.

As used herein, the term "diabody" means that the VH and VL domains thereof are expressed on a single polypeptide chain, but the linker used is too short to allow pairing between two domains of the same chain, thereby forcing the domains to pair with the complementary domains of the other chain and generating two antigen-binding sites (see, e.g., Holliger P. et al., Proc. Natl. Acad. Sci. USA 90: 6444-6448 (1993), and Poljak R. J. et al., Structure 2: 1121-1123 (1994)).

The above antibody fragments all retain the ability to specifically bind to the same antigen to which the full-length antibody binds, and/or compete with the full-length antibody for specific binding to an antigen. Herein, those skilled in the art may obtain an antigen-binding fragment of an antibody (e.g., the antibody fragment described above) from a given antibody (e.g., the antibody provided herein) using known conventional techniques and specifically screen for antigen-binding fragments of the antibody in the same manner as for an intact antibody.

As used herein, the term "multispecific antibody" refers to an antibody having a variety of different antigen-binding specificities, including, for example: bispecific antibodies, trispecific antibodies and tetraspecific antibodies. A "bispecific antibody" refers to an antibody having two different antigen-binding specificities, which is a conjugate formed by a first antibody (or a fragment thereof) and a second antibody (or a fragment thereof) or an antibody analog via a conjugating arm; the conjugating methods include, but are not limited to, chemical reaction, genetic fusion and enzymatic reaction. "Multispecific antibodies" include, for example, trispecific antibodies and tetraspecific antibodies, where a trispecific antibody is an antibody with three distinct antigen-binding specificities, and a tetraspecific antibody is an antibody with four distinct antigen-binding specificities.

As used herein, the terms "monoclonal antibody", "monoclonal Ab" and "mAb" have the same meaning and are used interchangeably. They refer to an antibody or a fragment of the antibody from a population of highly homologous antibody molecules, i.e., a population of antibody molecules that are identical except for possible naturally occurring spontaneous mutations. The monoclonal antibody is highly specific for a single epitope on an antigen. Relative to a monoclonal antibody, a polyclonal antibody generally comprises at least 2 or more different antibodies, and these different antibodies generally recognize different epitopes on an antigen. Moreover, the modifier "monoclonal" simply indicates that the antibody is characterized by being derived from a highly homologous population of antibodies and cannot be understood as requiring the production of the antibody by any particular method.

As used herein, the term "chimeric antibody" refers to an antibody in which a portion of the light chain or/and heavy chain is derived from one antibody (which may be derived from a particular species or belong to a particular antibody class or subclass), and another portion of the light chain or/and heavy chain is derived from another antibody (which may be derived from the same or different species or belong to the same or different antibody class or subclass), but which antibody nevertheless retains binding activity to a target antigen. For example, the term "chimeric antibody" may include antibodies in which the heavy and light chain variable regions are derived from a first antibody (e.g., of human origin) and the heavy and light chain constant regions are derived from a second antibody (e.g., of murine origin).

As used herein, the term "humanized antibody" refers to an antibody that can be prepared by replacing a portion of a human antibody with a portion of a non-human antibody that is prepared by immunizing a mammal other than a human. Typically, all or part of the CDR regions of a humanized antibody are derived from a non-human antibody (donor antibody), and all or part of the non-CDR regions (e.g., variable region FR and/or constant region) are derived from a human immunoglobulin (receptor antibody). For example, humanized antibodies can be prepared by grafting CDR sequences derived from germlines of other mammalian species onto human framework sequences.

As used herein, the term "variant", in the context of polypeptides (including polypeptides), also refers to a polypeptide or peptide comprising an amino acid sequence that has been altered by the introduction of amino acid residue substitutions, deletions or additions. In some cases, the term "variant" also refers to a polypeptide or peptide that has been modified (i.e., by covalently linking any type of molecule to a polypeptide or peptide). For example, but not by way of limitation, a polypeptide may be modified, e.g., by glycosylation, acetylation, pegylation, phosphorylation, amidation, derivatization by known protecting/blocking groups, proteolytic cleavage, attachment to cellular ligands or other proteins, etc. Derivatized polypeptides or peptides can be produced by chemical modification using techniques known to those skilled in the art, including but not limited to specific chemical cleavage, acetylation, formylation, and metabolic synthesis with tunicamycin. In addition, a variant has a function similar to, the same as, or improved compared to that of the polypeptide or peptide from which it is derived.

As used herein, the term "specific binding" refers to a non-random binding reaction between two molecules, such as a reaction between an antibody and its corresponding antigen. The strength or affinity of a specific binding interaction can be expressed as the equilibrium dissociation constant (KD) or the half-maximal effect concentration (EC₅₀) of the interaction.

Specific binding properties between two molecules can be determined using methods well known in the art. One of the methods involves measuring the rate of formation and dissociation of the antigen-binding site/antigen complex. Both the "association rate constant" (ka or kon ) and the "dissociation rate constant" (kdis or koff) can be calculated from the concentration and the actual rates of association and dissociation (see Malmqvist M, Nature, 1993, 361: 186-187). The ratio kdis/kon is equal to the dissociation constant KD (see Davies et al., Annual Rev Biochem, 1990; 59: 439-473). The values of KD, kon, and kdis can be measured using any valid method. In certain embodiments, the dissociation constant can be measured using bioluminescence interferometry (e.g., the ForteBio Octet method). In addition, the dissociation constant can also be measured using surface plasmon resonance techniques (such as Biacore) or Kinexa.

As used herein, the term "vector" refers to a nucleic acid delivery vehicle into which a polynucleotide can be inserted. When the vector allows for the expression of the protein encoded by the inserted polynucleotide, the vector is called an expression vector. A vector can be introduced into a host cell by transformation, transduction or transfection, and the genetic substance elements carried thereby can be expressed in the host cell. Vectors are well known to those skilled in the art, and include, but are not limited to: plasmids; phagemids; cosmids; artificial chromosomes, such as yeast artificial chromosomes (YACs), bacterial artificial chromosomes (BACs) or P1-derived artificial chromosomes (PACs); and phages such as a λ phage or M13 phage and animal viruses. The animal viruses that can be used as vectors include, but are not limited to retroviruses (including lentiviruses), adenoviruses, adeno-associated viruses, herpes viruses (e.g., herpes simplex virus), poxviruses, baculoviruses, papillomaviruses, and papovaviruses (such as SV40). A vector may comprise a variety of elements that control expression, including but not limited to a promoter sequence, a transcription initiation sequence, an enhancer sequence, a selection element and a reporter gene. In addition, the vector may further comprise an origin of replication.

Expression and cloning vectors comprise nucleic acid sequences that allow the vectors to replicate in one or more selected host cells. Typically, in a cloning vector, this sequence is one that enables the vector to replicate independently of host chromosomal DNA and includes an origin of replication or an autonomously replicating sequence. As used herein, the term "expression vector" refers to a vector comprising a recombinant polynucleotide, which comprises an expression control sequence operably linked to a nucleotide sequence to be expressed. The expression vector comprises sufficient cis-acting elements for expression; other elements for expression can be provided by a host cell or in an *in-vitro* expression system. Expression vectors include all those known in the art, such as cosmids, plasmids (e.g., naked or contained in liposomes) and viruses (e.g., lentiviruses, retroviruses, adenoviruses and adeno-associated viruses).

As used herein, the term "host cell" refers to a cell that can be used for vector introduction, including, but not limited to, prokaryotic cells such as *Escherichia coli* or *Bacillus subtilis*, fungal cells such as yeast cells or *Aspergillus*, insect cells such as S2 drosophila cells or Sf9, or animal cells such as fibroblasts, NS0 cells, Vero cells, Hela cells, COS cells, CHO cells (e.g., CHO-K1, CHO-S, CHO DXB11, ExpiCHO, CHO DG44 or CHO-EBNA cells), ExpiCHO cells, HEK293 cells, Expi293 cells, BHK cells and MDCKII cells.

As used herein, the term "identity" is used to refer to a sequence match between two polypeptides or between two nucleic acids. When a position in each of the two sequences being compared is occupied by the same base or amino acid monomer subunit (e.g., a position in each of the two DNA molecules is occupied by adenine, or a position in each of the two polypeptides is occupied by lysine), the molecules are identical at that position. The "percent identity" between two sequences is a function of the number of matching positions shared by the two sequences divided by the number of positions compared × 100. For example, if 6 out of 10 positions in two sequences match, the two sequences have 60% identity. For example, the DNA sequences CTGACT and CAGGTT share 50% identity (3 out of a total of 6 positions match). Generally, the comparison is performed when two sequences are aligned to produce maximum identity. Such alignments can be made by using, for example, the method described in Needleman et al. (1970) J. Mol. Biol. 48: 443-453, which is conveniently implemented via computer programs such as the Align program (DNAstar, Inc.). The percent identity between two amino acid sequences can also be determined using the algorithm by E. Meyers and W. Miller (Comput. Appl. Biosci., 4: 11-17 (1988)) incorporated into the ALIGN program (version 2.0), with the use of a PAM120 weight residue table, a gap length penalty of 12, and a gap penalty of 4. In addition, the percent identity between two amino acid sequences can be determined using the algorithm by Needleman and Wunsch (J MoI Biol. 48: 444-453 (1970)) incorporated into the GAP program of the GCG software package (available at www.gcg.com), with the use of a Blossum 62 matrix or a PAM250 matrix, and a gap weight of 16, 14, 12, 10, 8, 6 or 4 and a length weight of 1, 2, 3, 4, 5 or 6.

As used herein, the term "conservative substitution" refers to an amino acid substitution that does not adversely affect or alter the intended properties of a protein/polypeptide comprising an amino acid sequence. For example, conservative substitutions can be introduced by standard techniques known in the art, such as site-directed mutagenesis and PCR-mediated mutagenesis. Conservative amino acid substitutions include the replacement of an amino acid residue with an amino acid residue having a similar side chain, for example, substitution with a residue that is physically or functionally similar to the corresponding amino acid residue (e.g., having similar size, shape, charge, chemical properties, including the ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine) and aromatic side chains (e.g., tyrosine, phenylalanine, tryptophan, and histidine). Therefore, it is preferable to replace the corresponding amino acid residue with another amino acid residue from the same side chain family. Methods for identifying conservative amino acid substitutions are well known in the art (see, e.g., Brummell et al., Biochem. 32: 1180-1187 (1993); Kobayashi et al. Protein Eng. 12 (10): 879-884 (1999); and Burks et al. Proc. Natl Acad. Set USA 94: 412-417 (1997), which are incorporated herein by reference).

The designations of the twenty conventional amino acids referred to herein follow conventional usage. See, e.g., Immunology-A Synthesis (2nd Edition, E. S. Golub and D. R. Gren, Eds., Sinauer Associates, Sunderland, Mass. (1991)), which is incorporated herein by reference. In the present disclosure, the terms "polypeptide" and "protein" have the same meaning and are used interchangeably. Moreover, in the present disclosure, amino acids are generally represented by single-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to: a pH regulator, a surfactant, an adjuvant, an ion strength enhancer, a diluent, a reagent for maintaining the osmotic pressure, an absorption delaying agent, and a preservative. For example, the pH regulator includes, but is not limited to, a phosphate buffer. The surfactant includes, but is not limited to, a cationic, anionic or nonionic surfactant, such as Tween-80. The ionic strength enhancer includes, but is not limited to, sodium chloride. The preservative includes, but is not limited to, various antibacterial reagents and antifungal reagents, such as parabens, chlorobutanol, phenol, and sorbic acid. The reagent for maintaining osmotic pressure includes, but is not limited to, a saccharide, NaCl and an analog thereof. The absorption delaying agent includes, but is not limited to, monostearate and gelatin. The diluent includes, but is not limited to, water, aqueous buffers (such as buffered saline), alcohols, polyols (such as glycerol), etc. The preservative includes, but is not limited to, various antibacterial reagents and antifungal reagents, such as thiomersal, 2-phenoxyethanol, parabens, chlorobutanol, phenol and sorbic acid. The "stabilizer" has the meaning that is commonly understood by those skilled in the art and can stabilize the desired activity of active ingredients in a drug. The stabilizer includes, but is not limited to, sodium glutamate, gelatin, SPGA, saccharides (such as sorbitol, mannitol, starch, sucrose, lactose, dextran or glucose), amino acids (such as glutamic acid or glycine), proteins (such as dry whey, albumin or casein), or degradation products thereof (such as lactalbumin hydrolysate).

As used herein, the term "prevention" refers to a method performed to prevent or delay the onset of a disease or disorder or symptom (e.g., a tumor) in a subject. As used herein, the term "treatment" refers to a method performed to obtain beneficial or desired clinical outcomes. For the purpose of the present disclosure, beneficial or desired clinical outcomes include, but are not limited to, alleviation of symptoms, reduction in the extent of the disease, stabilization of the disease state (i.e., no longer worsening), delay or slowing of disease progression, improvement or alleviation of the disease state, and remission of symptoms (whether partial or complete), whether detectable or undetectable. In addition, "treatment" may also refer to extending the survival period compared to the expected survival period (if no treatment is administered).

As used herein, the term "subject" refers to a mammal, e.g., a primate mammal, e.g., a human. In certain embodiments, the subject (e.g., a human) suffers from a tumor, or is at risk of suffering from such a disease.

As used herein, the term "effective amount" refers to an amount sufficient to obtain or at least partially obtain the desired effect. For example, an effective amount for preventing a disease (e.g., a tumor) refers to an amount sufficient to prevent, inhibit or delay the onset of a disease (e.g., a tumor); an effective amount for treating a disease refers to an amount sufficient to cure or at least partially inhibit a disease and complications thereof in a patient suffering from the disease. It is well within the competence of those skilled in the art to determine such effective amounts. For example, an amount effective for therapeutic use will depend on the severity of the disease to be treated, the overall status of the patient's own immune system, the general situation of the patient such as age, body weight and gender, the mode of administration of the drug, other concurrent treatments, etc.

As used herein, the term "effector function" refers to those biological activities attributable to the Fc region of an antibody (either a native-sequence Fc region or an amino acid sequence-variant Fc region), and these activities vary with the antibody isotype. Examples of antibody effector functions include, but are not limited to: Fc receptor binding affinity, antibody-dependent cell-mediated cytotoxicity (ADCC), complement-dependent cytotoxicity (CDC), antibody-dependent cell phagocytosis (ADCP), down-regulation of cell surface receptors (such as B cell receptors), B cell activation, cytokine secretion, half-life/clearance of antibodies and antigen-antibody complexes, etc. Methods for altering the effector functions of antibodies are known in the art, for example, by introducing mutations in the Fc region.

As used herein, the term "antibody-dependent cell-mediated cytotoxicity (ADCC) " refers to a form of cytotoxicity in which Ig binds to an Fc receptor (FcR) present on cytotoxic cells (e.g., natural killer (NK) cells, neutrophils or macrophages), enabling these cytotoxic effector cells to specifically bind to antigen-attached target cells and subsequently kill target cells by secreting cytotoxins.

As used herein, the term "complement-dependent cytotoxicity (CDC) " refers to a complement-mediated cytotoxic effect in which a specific antibody binds to a corresponding antigen on the cell membrane surface to form a complex, which activates the classical pathway of complement; the membrane attack complex formed thereby exerts a lytic effect on a target cell.

Herein, "combination" includes therapies that can be administered separately, e.g., formulated separately for individual administration (e.g., may be provided in a kit), and therapies that can be administered together as a single formulation (i.e., "co-formulation"). In certain embodiments, the anti-ADAM9 antibodies or the antigen-binding fragments thereof of the present disclosure may be administered sequentially. In other embodiments, the anti-ADAM9 antibodies or the antigen-binding fragments thereof may be administered simultaneously. The antibodies or the antigen-binding fragments thereof of the present disclosure may be used in combination with at least one other (active) agent in any manner.

In such combination therapies, various active agents often have different complementary mechanisms of action, and combination therapies may result in a synergistic effect. A combination therapy comprises therapeutic agents that affect immune responses (e.g., enhance or activate responses) and therapeutic agents that affect (e.g., inhibit or kill) tumor/cancer cells. A combination therapy can reduce the likelihood of drug-resistant cancer cell development. A combination therapy allows for a reduction in the dose of one or more of the agents to reduce or eliminate the adverse effects associated with one or more of the agents. Such combination therapies may have a synergistic therapeutic or prophylactic effect on the underlying diseases, disorders or conditions.

Herein, ADAM9 positivity is determined by immunohistochemistry and evaluation of staining intensity performed by professional clinical pathologists.

The terms "cancer" and "tumor" are used interchangeably and refer to a broad category of diseases characterized by uncontrolled growth of abnormal cells in the body. Unregulated cell division may result in the formation of malignant tumors or cells invading adjacent tissues, and may be transferred to distal parts of the body through the lymphatic system or bloodstream. Cancer includes benign and malignant cancers, as well as dormant tumors or micrometastases. Cancer also includes hematological malignancies.

The embodiments of the present disclosure will be described in detail below in conjunction with examples. However, it will be understood by those skilled in the art that the following examples are only used to illustrate the present disclosure and are not intended to limit the scope of the present disclosure. Various objectives and advantageous aspects of the present disclosure will become implementable to those skilled in the art according to the following detailed description of the accompanying drawings and preferred embodiments.

### Brief Description of the Drawings

FIG. 1: Results of affinity detection of anti-human ADAM9 murine antibodies for EBC-1 cells.
FIG. 2: Results of affinity detection of anti-human ADAM9 chimeric and humanized antibodies for EBC-1 cells.
FIG. 3: Results of affinity detection of anti-human ADAM9 chimeric and humanized antibodies for HCC1806 cells.
FIG. 4: Results of endocytic activity detection of anti-human ADAM9 chimeric and humanized antibodies.
FIG. 5: Results of specificity detection of anti-human ADAM9 humanized antibodies.

### Sequence information

The information of the sequences to which the present disclosure relates is described in the following table:

**Table 1: Sequence description**

| SEQ ID NO: | Sequence description | Amino acid/nucleotide sequence |
|---|---|---|
| 1 | Amino acid sequence of Ab01 VH | QAYLQQSGAELVRPGASVKMSCKASGYTFTNYNVH WVKQTPRQGLEWIGAIYPGNGDTSYNQKFKGKATLT VDKSSSTAYMQLSSLTSEDSAVYFCAIGYSYFDYWG QGTTLTVSS |
| 2 | Amino acid sequence of Ab01 VL | DIVLTQSPATLSVTPGDSVSLSCRASQSISNNLHWYQ QKSHESPRLLIKFASQSISGIPSKFSGSGSGTDFTLSINS VETEDFGMYFCQQSYSWYTFGSGTKLEIK |
| 3 | Amino acid sequence of Ab01-HZ43 VH | QVQLVQSGAEVKKPGASVKVSCKASGYTFTNYNVH WVRQAPGQGLEWIGAIYPGNGDTSYNQKFKGRVTM TVDTSTSTVYMELSSLRSEDTAVYYCARGYSYFDYW GQGTLVTVSS |
| 4 | Amino acid sequence of Ab01-HZ43 VL | DIVLTQSPATLSVSPGERATLSCRASQSISNNLHWYQ QKPGQAPRLLIKFASQSISGIPARFSGSGSGTEFTLTISS VQSEDFAVYYCQQSYSWYTFGQGTKLEIK |
| 5 | Amino acid sequence of Ab01-HZ43/Ab02-HZ42/Ab03-HZ43/Ab04-HZ81 CH | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPEAAGAPSVFLFPPKPKDTLMISRTPEVTCVVVD VSHEDPEVKFNWYVDGVEVHNAKTKPREEQYNSTY RVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKL TVDKSRWQQGNVFSCSVMHEALHNHYTQKSLSLSP GK |
| 6 | Amino acid sequence of Ab01-HZ43/Ab02-HZ42/Ab03-HZ43/Ab04-HZ81 CL | |
| 7 | Amino acid sequence of Ab02 VH | QVQLQQPGAELVRPGTSVKLSCKASGYTFTSYWMH WVKQRPGQGLEWIGLIDPSDTYTNYNQNFKGKATLT VDTSSRIVYMQLSSLTSEDSAVYYCARGDYGNNVGY FDVWGAGTTVTVSP |
| 8 | Amino acid sequence of Ab02 VL | DIQMIQSPASLSASVGETVTITCRASENIDSYLAWYQQ KQGKSPQLLVYVATLLADGVPSRFSGSGSGTQYSLKI NSLQSEDVARYYCQHYYITPWTFGGGTKLEIK |
| 9 | Amino acid sequence of Ab02-HZ42 VH | QVQLVQSGAEVVKPGASVKLSCKASGYTFTNYWMH WVRQRPGQGLEWIGLIDASDSYINYNAKFQGRVTITV DTSTSTAYMELSSLRSEDTAVYYCARGDYGNNVGYF DVWGQGTLVTVSS |
| 10 | Amino acid sequence of Ab02-HZ42 VL | DIQMTQSPSSLSASVGQRVTITCRASENIDRYLAWYQ QKQGKAPKLLVYVATLLASGVPSRFSGSGSGTDYTL TISSLQPEDFATYYCQHYYITPWTFGQGTKLEIK |
| 11 | Amino acid sequence of Ab01/Ab01-HZ43/Ab02-HZ42 Chothia CDR H1 | GYTFTNY |
| 12 | Amino acid sequence of Ab01/Ab01-HZ43 Chothia CDR H2 | YPGNGD |
| 13 | Amino acid sequence of Ab01/Ab01-HZ43 Chothia/Abm/Kaba t CDR H3 | GYSYFDY |
| 14 | Amino acid sequence of Ab01/Ab01-HZ43 Chothia/Abm/Kaba t CDR L1 | RASQSISNNLH |
| 15 | Amino acid sequence of Ab01/Ab01-HZ43 Chothia/Abm/Kaba t CDR L2 | FASQSIS |
| 16 | Amino acid sequence of Ab01/Ab01-HZ43 Chothia/Abm/Kaba t/IMGT CDR L3 | QQSYSWYT |
| 17 | Amino acid sequence of Ab01/Ab01-HZ43 Abm CDR H1 | GYTFTNYNVH |
| 18 | Amino acid sequence of Ab01/Ab01-HZ43 Abm CDR H2 | AIYPGNGDTS |
| 19 | Amino acid sequence of Ab01/Ab01-HZ43 Kabat CDR H1 | NYNVH |
| 20 | Amino acid sequence of Ab01/Ab01-HZ43 Kabat CDR H2 | AIYPGNGDTSYNQKFKG |
| 21 | Amino acid sequence of Ab01/Ab01-HZ43 IMGT CDR H1 | GYTFTNYN |
| 22 | Amino acid sequence of Ab01/Ab01-HZ43 IMGT CDR H2 | IYPGNGDT |
| 23 | Amino acid sequence of Ab01-HZ43 IMGT CDR H3 | ARGYSYFDY |
| 24 | Amino acid sequence of Ab01/Ab01-HZ43 IMGT CDR L1 | QSISNN |
| 25 | Amino acid sequence of Ab01/Ab01-HZ43 IMGT CDR L2 | FAS |
| 26 | Amino acid sequence of Ab02-HZ42 Chothia CDR H2 | DASDSY |
| 27 | Amino acid sequence of Ab02/Ab02-HZ42 Chothia/Abm/Kaba t CDR H3 | GDYGNNVGYFDV |
| 28 | Amino acid sequence of Ab02-HZ42 Chothia/Abm/Kaba t CDR L1 | RASENIDRYLA |
| 29 | Amino acid sequence of Ab02-HZ42 Chothia/Abm/Kaba t CDR L2 | VATLLAS |
| 30 | Amino acid sequence of Ab02/Ab02-HZ42 Chothia/Abm/Kaba t/IMGT CDR L3 | QHYYITPWT |
| 31 | Amino acid sequence of Ab02-HZ42 Abm CDR H1 | GYTFTNYWMH |
| 32 | Amino acid sequence of Ab02-HZ42 Abm CDR H2 | LIDASDSYIN |
| 33 | Amino acid sequence of Ab02-HZ42 Kabat CDR H1 | NYWMH |
| 34 | Amino acid sequence of Ab02-HZ42 Kabat CDR H2 | LIDASDSYINYNAKFQG |
| 35 | Amino acid sequence of Ab02-HZ42 IMGT CDR H1 | GYTFTNYW |
| 36 | Amino acid sequence of Ab02-HZ42 IMGT CDR H2 | IDASDSYI |
| 37 | Amino acid sequence of Ab02/Ab02-HZ42 IMGT CDR H3 | ARGDYGNNVGYFDV |
| 38 | Amino acid sequence of Ab02-HZ42 IMGT CDR L1 | ENIDRY |
| 39 | Amino acid sequence of Ab02/Ab02-HZ42 IMGT CDR L2 | VA |
| 40 | Amino acid sequence of Ab01-HZ43 HC | QVQLVQSGAEVKKPGASVKVSCKASGYTFTNYNVH WVRQAPGQGLEWIGAIYPGNGDTSYNQKFKGRVTM TVDTSTSTVYMELSSLRSEDTAVYYCARGYSYFDYW GQGTLVTVSSASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKKVEPK SCDKTHTCPPCPAPEAAGAPSVFLFPPKPKDTLMISRT PEVTCVVVDVSHEDPEVKFNWYVDGVEVHNAKTKP REEQYNSTYRVVSVLTVLHQDWLNGKEYKCKVSNK ALPAPIEKTISKAKGQPREPQVYTLPPSRDELTKNQVS LTCLVKGFYPSDIAVEWESNGQPENNYKTTPPVLDSD GSFFLYSKLTVDKSRWQQGNVFSCSVMHEALHNHY TQKSLSLSPGK |
| 41 | Amino acid sequence of Ab01-HZ43 LC | DIVLTQSPATLSVSPGERATLSCRASQSISNNLHWYQ QKPGQAPRLLIKFASQSISGIPARFSGSGSGTEFTLTISS VQSEDFAVYYCQQSYSWYTFGQGTKLEIKRTVAAPS VFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGEC |
| 42 | Amino acid sequence of Ab02-HZ42 HC | QVQLVQSGAEVVKPGASVKLSCKASGYTFTNYWMH WVRQRPGQGLEWIGLIDASDSYINYNAKFQGRVTITV DTSTSTAYMELSSLRSEDTAVYYCARGDYGNNVGYF DVWGQGTLVTVSSASTKGPSVFPLAPSSKSTSGGTAA LGCLVKDYFPEPVTVSWNSGALTSGVHTFPAVLQSS GLYSLSSVVTVPSSSLGTQTYICNVNHKPSNTKVDKK VEPKSCDKTHTCPPCPAPEAAGAPSVFLFPPKPKDTL MISRTPEVTCVVVDVSHEDPEVKFNWYVDGVEVHN AKTKPREEQYNSTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTP PVLDSDGSFFLYSKLTVDKSRWQQGNVFSCSVMHEA LHNHYTQKSLSLSPGK |
| 43 | Amino acid sequence of Ab02-HZ42 LC | DIQMTQSPSSLSASVGQRVTITCRASENIDRYLAWYQ QKQGKAPKLLVYVATLLASGVPSRFSGSGSGTDYTL TISSLQPEDFATYYCQHYYITPWTFGQGTKLEIKRTV AAPSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC |
| 44 | Nucleotide sequence of Ab01-HZ43 VH | CAAGTACAACTCGTGCAAAGCGGCGCAGAAGTAA AGAAGCCTGGTGCCTCTGTGAAAGTGTCATGCAAG GCCTCCGGTTACACTTTCACCAACTACAACGTACA TTGGGTTCGGCAGGCTCCAGGACAAGGACTGGAAT GGATCGGAGCCATTTACCCCGGAAACGGGGATACC TCCTACAATCAGAAGTTCAAAGGTAGGGTGACTAT GACCGTCGACACCTCTACGAGCACGGTGTATATGG AGTTGTCATCTCTGCGGAGCGAAGATACTGCTGTG TATTATTGTGCAAGGGGATACTCTTACTTCGATTAC TGGGGGCAAGGAACACTCGTAACGGTGTCCAGC |
| 45 | Nucleotide sequence of Ab01-HZ43 VL | GACATCGTCCTTACCCAGTCACCCGCCACTCTTTCC GTAAGCCCTGGAGAGAGAGCTACGCTTTCCTGTAG AGCCTCCCAGTCCATTTCAAACAACCTGCACTGGT ATCAACAGAAACCAGGACAGGCCCCTAGATTGCTG ATCAAATTCGCTAGCCAATCTATCTCTGGGATACCT GCTCGCTTCAGTGGATCCGGTTCTGGTACAGAGTT CACCCTCACCATCAGCTCTGTCCAGTCAGAAGACT TCGCCGTGTACTACTGCCAGCAGTCATACTCTTGGT ACACCTTCGGCCAGGGGACTAAGCTCGAAATCAAG |
| 46 | Nucleotide sequence of Ab02-HZ42 VH | CAAGTCCAGCTGGTGCAAAGCGGAGCAGAAGTAG TAAAGCCGGGTGCTAGTGTCAAACTGTCTTGTAAG GCTTCTGGGTACACTTTCACGAATTACTGGATGCAT TGGGTCAGGCAGAGACCCGGACAAGGGCTTGAAT GGATCGGACTGATTGATGCTAGCGACAGCTACATC AACTACAACGCTAAGTTTCAGGGGAGGGTTACGAT CACCGTCGATACCAGCACCAGCACAGCCTACATGG AGCTCTCTTCATTGAGATCTGAGGATACAGCAGTC TACTACTGTGCCAGAGGCGATTATGGTAACAACGT AGGGTATTTTGACGTCTGGGGTCAGGGTACTCTGG TGACTGTCAGTAGC |
| 47 | Nucleotide sequence of Ab02-HZ42 VL | GATATCCAGATGACACAGTCCCCGAGTTCCCTCTC CGCTAGCGTAGGACAAAGGGTGACTATTACATGCC GCGCCAGCGAGAATATTGACAGGTATCTCGCCTGG TATCAGCAGAAACAGGGTAAGGCCCCTAAGTTGTT GGTGTACGTTGCCACTCTTCTGGCTTCTGGTGTTCC TAGCAGATTCTCCGGTAGCGGTAGTGGTACCGATT ACACTCTGACAATCTCCTCATTGCAGCCGGAAGAT TTTGCCACATACTATTGCCAGCACTACTACATAAC ACCGTGGACCTTTGGCCAGGGAACCAAATTGGAGA TCAAG |
| 48 | Amino acid sequence of Ab03 VH | QVQLLQPGAELVKPGTSVKLSCKASGYTFTTYWMH WVKQRPGRGLEWIGRFDPNSGSSKYNEKFKSKATLT VDKPSSTAYMQLNSLTSEDSAVYYCATDTYDYFDY WGQGTTLTVSS |
| 49 | Amino acid sequence of Ab03 VL | ETTVTQSPASLSMAIGEKVTIRCITSTDIDDDMSWYQ QKAGEPPKLLVSDGNTLRPGVPSRFSASGYGTDFVFT IENMLSEDVADYYCLQSENLPYTFGGGTKLEIK |
| 50 | Amino acid sequence of Ab03-HZ43 VH | QVQLVQSGAEVKKPGASVKVSCKASGYTFTTYWMH WVRQAPGQGLEWIGRFDPNSGSSKYNEKFKSRVTLT VDTSISTAYMELSRLRSDDTAVYYCATDTYDYFDYW GQGTLVTVSS |
| 51 | Amino acid sequence of Ab03-HZ43 VL | ETTLTQSPAFMSATPGDKVNISCITSTDIDDDMSWYQ QKPGEPPILLVSDGNTLRPGIPPRFSGSGYGTDFTLTIN NMESEDAATYYCLQSENLPYTFGGGTKLEIK |
| 52 | Amino acid sequence of Ab04 VH | DVQLQESGPGLVKPSQSLSLTCSVTGYSITSGYFWNW IRQFPGNKLEWMGYISYDGSNNYNPSLKSRISITRDTS KNQFFLKLNSVTTEDTATYYCTRSDAAYYDYDVGW GQGTLVTVSA |
| 53 | Amino acid sequence of Ab04 VL | DIQMTQSPASLSASVGETVTITCRASENIDSYLAWYQ QKQGKSPQLLVYAATLLADGVPSRFSGSGSGTQYSL KINSLQSEDVARYYCQHYYITPLTFGAGTKLELK |
| 54 | Amino acid sequence of Ab04-HZ81 VH | QVQLQESGPGLVKPSQTLSLTCTVSGYSITSGYFWNW IRQFPGKGLEWMGYISYDGSNNYNPSLKSRITISRDTS KNQFSLKLNSVTAADTAVYYCTRSDAAYYDYDVGW GQGTLVTVSS |
| 55 | Amino acid sequence of Ab04-HZ81 VL | DIQMTQSPSSLSASVGDRVTITCRASENIDSYLAWYQ QKPGKAPKLLIYAATLLADGVPSRFSGSGSGTDFTLTI NSLQPEDVATYYCQHYYITPLTFGQGTKLEIK |
| 56 | Amino acid sequence of Ab03/Ab03-HZ43 Chothia CDR H1 | GYTFTTY |
| 57 | Amino acid sequence of Ab03/Ab03-HZ43 Chothia CDR H2 | DPNSGS |
| 58 | Amino acid sequence of Ab03/Ab03-HZ43 Chothia CDR H3 | DTYDYFDY |
| 59 | Amino acid sequence of Ab03/Ab03-HZ43 Chothia/Kabat/ Abm CDR L1 | ITSTDIDDDMS |
| 60 | Amino acid sequence of Ab03/Ab03-HZ43 Chothia/Kabat/ Abm CDR L2 | DGNTLRP |
| 61 | Amino acid sequence of Ab03/Ab03-HZ43 Chothia/Kabat/ Abm CDR L3 | LQSENLPYT |
| 62 | Amino acid sequence of Ab03/Ab03-HZ43 Abm/Kabat CDR H1 | TYWMH |
| 63 | Amino acid sequence of Ab03/Ab03-HZ43 Abm/Kabat CDR H2 | RFDPNSGSSKYNEKFKS |
| 64 | Amino acid sequence of Ab03/Ab03-HZ43 Abm/Kabat CDR H3 | DTYDYFDY |
| 65 | Amino acid sequence of Ab03/Ab03-HZ43 IMGT CDR H1 | GYTFTTYW |
| 66 | Amino acid sequence of Ab03/Ab03-HZ43 IMGT CDR H2 | FDPNSGSS |
| 67 | Amino acid sequence of Ab03/Ab03-HZ43 IMGT CDR H3 | ATDTYDYFDY |
| 68 | Amino acid sequence of Ab03/Ab03-HZ43 IMGT CDR L1 | TDIDDD |
| 69 | Amino acid sequence of Ab03/Ab03-HZ43 IMGT CDR L2 | DGN |
| 70 | Amino acid sequence of Ab03/Ab03-HZ43 IMGT CDR L3 | LQSENLPYT |
| 71 | Amino acid sequence of Ab04/Ab04-HZ81 Chothia CDR H1 | GYSITSGY |
| 72 | Amino acid sequence of Ab04/Ab04-HZ81 Chothia CDR H2 | SYDGS |
| 73 | Amino acid sequence of Ab04/Ab04-HZ81 Chothia/Kabat/ Abm CDR H3 | SDAAYYDYDVG |
| 74 | Amino acid sequence of Ab04/Ab04-HZ81 Chothia/Kabat/ Abm CDR L1 | RASENIDSYLA |
| 75 | Amino acid sequence of Ab04/Ab04-HZ81 Chothia/Kabat/ Abm CDR L2 | AATLLAD |
| 76 | Amino acid sequence of Ab04/Ab04-HZ81 Chothia/Kabat/ Abm/IMGT CDR L3 | QHYYITPLT |
| 77 | Amino acid sequence of Ab04/Ab04-HZ81 Abm CDR H1 | GYSITSGYFWN |
| 78 | Amino acid sequence of Ab04/Ab04-HZ81 Abm CDR H2 | YISYDGSNN |
| 79 | Amino acid sequence of Ab04/Ab04-HZ81 Kabat CDR H1 | SGYFWN |
| 80 | Amino acid sequence of Ab04/Ab04-HZ81 Kabat CDR H2 | YISYDGSNNYNPSLKS |
| 81 | Amino acid sequence of Ab04/Ab04-HZ81 IMGT CDR H1 | GYSITSGYF |
| 82 | Amino acid sequence of Ab04/Ab04-HZ81 IMGT CDR H2 | ISYDGSN |
| 83 | Amino acid sequence of Ab04/Ab04-HZ81 IMGT CDR H3 | TRSDAAYYDYDVG |
| 84 | Amino acid sequence of Ab04/Ab04-HZ81 IMGT CDR L1 | ENIDSY |
| 85 | Amino acid sequence of Ab04/Ab04-HZ81 IMGT CDR L2 | AAT |
| 86 | Amino acid sequence of Ab03-HZ43 HC | QVQLVQSGAEVKKPGASVKVSCKASGYTFTTYWMH WVRQAPGQGLEWIGRFDPNSGSSKYNEKFKSRVTLT VDTSISTAYMELSRLRSDDTAVYYCATDTYDYFDYW GQGTLVTVSSASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKKVEPK SCDKTHTCPPCPAPEAAGAPSVFLFPPKPKDTLMISRT PEVTCVVVDVSHEDPEVKFNWYVDGVEVHNAKTKP REEQYNSTYRVVSVLTVLHQDWLNGKEYKCKVSNK ALPAPIEKTISKAKGQPREPQVYTLPPSRDELTKNQVS LTCLVKGFYPSDIAVEWESNGQPENNYKTTPPVLDSD GSFFLYSKLTVDKSRWQQGNVFSCSVMHEALHNHY TQKSLSLSPGK |
| 87 | Amino acid sequence of Ab03-HZ43 LC | ETTLTQSPAFMSATPGDKVNISCITSTDIDDDMSWYQ QKPGEPPILLVSDGNTLRPGIPPRFSGSGYGTDFTLTIN NMESEDAATYYCLQSENLPYTFGGGTKLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| 88 | Amino acid sequence of Ab04-HZ81 HC | QVQLQESGPGLVKPSQTLSLTCTVSGYSITSGYFWNW IRQFPGKGLEWMGYISYDGSNNYNPSLKSRITISRDTS KNQFSLKLNSVTAADTAVYYCTRSDAAYYDYDVGW GQGTLVTVSSASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKKVEPK SCDKTHTCPPCPAPEAAGAPSVFLFPPKPKDTLMISRT PEVTCVVVDVSHEDPEVKFNWYVDGVEVHNAKTKP REEQYNSTYRVVSVLTVLHQDWLNGKEYKCKVSNK ALPAPIEKTISKAKGQPREPQVYTLPPSRDELTKNQVS LTCLVKGFYPSDIAVEWESNGQPENNYKTTPPVLDSD GSFFLYSKLTVDKSRWQQGNVFSCSVMHEALHNHY TQKSLSLSPGK |
| 89 | Amino acid sequence of Ab04-HZ81 LC | DIQMTQSPSSLSASVGDRVTITCRASENIDSYLAWYQ QKPGKAPKLLIYAATLLADGVPSRFSGSGSGTDFTLTI NSLQPEDVATYYCQHYYITPLTFGQGTKLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| 90 | Nucleotide sequence of Ab03-HZ43 VH | CAAGTGCAACTGGTGCAGAGTGGTGCCGAAGTGA AGAAACCAGGGGCCTCCGTCAAGGTCAGCTGTAAG GCATCCGGCTACACATTCACAACTTATTGGATGCA TTGGGTCAGACAAGCTCCTGGGCAGGGACTGGAAT GGATAGGTCGGTTCGATCCAAACTCCGGATCCAGC AAGTACAACGAGAAGTTCAAGAGCCGGGTGACAC TTACTGTCGACACCTCTATATCCACAGCCTATATGG AGCTCTCACGCCTGAGATCCGATGATACGGCCGTC TACTACTGCGCTACAGACACCTATGATTACTTCGA CTACTGGGGACAGGGGACACTGGTCACTGTGTCAT CA |
| 91 | Nucleotide sequence of Ab03-HZ43 VL | GAAACCACACTTACACAGTCACCTGCATTTATGTC AGCAACCCCAGGCGACAAAGTTAACATCAGCTGTA TTACTAGCACCGACATCGACGATGACATGTCCTGG TACCAGCAAAAGCCTGGTGAGCCTCCTATCCTCTT GGTTTCAGACGGCAATACTCTCAGACCAGGTATCC CACCTCGATTTTCCGGCTCTGGTTATGGGACAGATT TCACCCTGACGATCAACAACATGGAATCTGAAGAC GCTGCCACTTACTACTGTCTGCAGAGTGAGAATTT GCCTTACACCTTTGGTGGAGGAACTAAACTGGAAA TCAAA |
| 92 | Nucleotide sequence of Ab04-HZ81 VH | CAAGTACAGTTGCAAGAAAGCGGCCCTGGCCTGGT CAAGCCCTCTCAGACCCTGTCCCTGACCTGTACAG TCAGCGGATATTCCATAACCTCCGGGTACTTTTGG AACTGGATTAGGCAATTTCCAGGCAAAGGCCTGGA ATGGATGGGCTATATCTCATACGACGGTAGCAACA ATTATAATCCCAGTCTGAAAAGCCGGATTACCATC AGTCGGGACACTTCTAAGAACCAGTTCTCCCTCAA GTTGAATAGTGTCACCGCAGCCGATACAGCTGTCT ACTATTGTACTAGATCCGATGCCGCCTATTATGATT ACGACGTTGGCTGGGGTCAAGGAACTCTGGTGACG GTAAGTTCT |
| 93 | Nucleotide sequence of Ab04-HZ81 VL | GACATCCAGATGACCCAAAGCCCCTCAAGCCTTAG CGCCTCCGTTGGCGACAGAGTGACAATCACATGCC GCGCCTCTGAGAACATCGACTCCTATCTCGCATGG TATCAGCAAAAGCCAGGTAAGGCTCCCAAGCTTCT GATCTATGCTGCTACGCTGTTGGCTGATGGAGTGC CTAGCCGGTTTAGTGGAAGTGGTTCTGGCACAGAT TTCACACTGACCATAAACAGCCTGCAACCTGAAGA TGTAGCCACTTATTACTGCCAGCACTACTATATCAC GCCATTGACCTTCGGGCAGGGCACTAAGCTGGAAA TCAAA |
| 94 | Amino acid sequence of Ab01 IMGT CDR H3 | AIGYSYFDY |
| 95 | Amino acid sequence of Ab02 Chothia CDR H1 | GYTFTSY |
| 96 | Amino acid sequence of Ab02 Chothia CDR H2 | DPSDTY |
| 97 | Amino acid sequence of Ab02 Chothia/Abm/Kaba t CDR L1 | RASENIDSYLA |
| 98 | Amino acid sequence of Ab02 Chothia/Abm/Kaba t CDR L2 | VATLLAD |
| 99 | Amino acid sequence of Ab02 Abm CDR H1 | GYTFTSYWMH |
| 100 | Amino acid sequence of Ab02 Abm CDR H2 | LIDPSDTYTN |
| 101 | Amino acid sequence of Ab02 Kabat CDR H1 | SYWMH |
| 102 | Amino acid sequence of Ab02 Kabat CDR H2 | LIDPSDTYTNYNQNFKG |
| 103 | Amino acid sequence of Ab02 IMGT CDR H1 | GYTFTSYW |
| 104 | Amino acid sequence of Ab02 IMGT CDR H2 | IDPSDTYT |
| 105 | Amino acid sequence of Ab02 IMGT CDR L1 | ENIDSY |

### Detailed Description of Embodiments

The present disclosure is described with reference to the following examples, which are meant to illustrate the present disclosure (but not limit the present disclosure).

Unless otherwise indicated, molecular biology experimental methods and immunoassays used in the present disclosure are performed essentially with reference to methods described in J. Sambrook et al., Molecular Cloning: A Laboratory Manual, 2nd Edition, Cold Spring Harbor Laboratory Press, 1989, and F. M. Ausubel et al., Short Protocols In Molecular Biology, 3rd Edition, John Wiley & Sons, Inc., 1995. It is known to those skilled in the art that the examples illustrate the present disclosure by way of example and are not intended to limit the claimed scope of the present disclosure.

### Example 1: Preparation of ADAM9 antigen and control antibody protein

### 1.1 Preparation of ADAM9 antigen

The extracellular domain sequences of Human ADAM9 (UniProt: Q13443) and monkey ADAM9 (UniProt: A0A2K5X4X8) were gene-synthesized by GenScript, and then constructed into the pTT5 vector, with six histidines introduced at the C-terminus. The recombinant plasmids were extracted. After confirming correct sequencing, the plasmids were transiently transfected into HEK293 cells for transient expression. The cell supernatants were collected on day 6, and human ADAM9 protein and monkey ADAM9 protein were separately obtained through purification.

### 1.2 Expression of ADAM9 control antibody

The ADAM9 control antibody is derived from hMAB-A(2I .2) in Patent CN 201980049672. After codon optimization by GenScript, the heavy and light chain constant regions of the antibody are as set forth in SEQ ID NO: 5 and SEQ ID NO: 6 in the patent application, respectively. The nucleotide sequences of the antibody heavy and light chains were separately synthesized and cloned into the pTT5 vector. Then, the pTT5 plasmids corresponding to the nucleotides of the antibody heavy and light chains were co-transfected into CHOS cells. After the cell supernatant was collected by centrifugation, the antibodies in supernatant were purified using Protein A (MabSelect SuRe, GE) to obtain a control antibody protein.

### 1.3 Preparation of ADAM9 overexpressing cell line

According to the full-length proteins of human ADAM9 (NP_003807.1), monkey ADAM9 (XP_045253681.1), rat ADAM9 (NP_001014772.2) and mouse ADAM9 (NP_001257925.1), Nanjing GenScript Biotech Co., Ltd. was commissioned to perform codon optimization and gene synthesis. The genes were constructed into the lentiviral vector pLVX-IRES-puro, followed by virus packaging and infection of BaF3 cells. After pressure screening and verification by flow cytometry, overexpressing cell lines stably expressing human, monkey, rat and mouse ADAM9 were obtained.

### Example 2: Preparation of anti-human ADAM9 monoclonal antibody

### 2.1 Immunization of mice

Wild-type mice were immunized with the human ADAM9 protein prepared in section 1.1. Specifically, the first immunization was performed using Freund's complete adjuvant, and subsequent booster immunizations were administered with Freund's incomplete adjuvant at one-week intervals. During immunization, the serum titer of anti-ADAM9 antibodies was monitored every two weeks by protein ELISA and flow cytometry. The specific procedures for ELISA were as follows: Human ADAM9-his was diluted to 1 µg/ml in CBS coating solution, added at 100 µl/well, and then incubated overnight at 4°C. Washing was performed once with 300 µl of PBST, and 100 µl of PBS + 2%BSA was added for blocking at 37°C for 2 hours. The blocking solution was discarded. The serum was subjected to gradient dilution, starting in PBS + 2% BSA with a 100-fold dilution followed by 3-fold gradient dilutions, and then incubated at 37°C for 2 hours. Washing was performed 3 times with 300 µl of PBST. HRP-Goat-Anti-mouse IgG was diluted at a ratio of 1 : 10,000 in PBS (2% BSA). 100 µl of the diluted solution was added to the well plate and then incubated at 37°C for 1 hour. Washing was performed 5 times with 300 uL of PBST. 80 µl of TMB (Huzhou InnoReagents Co., Ltd.) was added. After color development, 40 µl of 2N H₂SO₄ was added to terminate the reaction, and the absorbance at 450 nm was read using a microplate reader. Flow cytometry: HCT116 cells were collected and washed 3 times with PBS. Mouse serum was gradiently diluted in PBS + 1% BSA solution, starting with a 50-fold dilution followed by 3-fold dilutions, resulting in a total of 11 concentration points. The volume per well was 50 µL. Simultaneously, blank cell wells and negative mouse serum control wells were set up. NCI-H226 cells were resuspended in PBS + 1% BSA solution at a density of 1 × 10⁵ cells/well. The cell suspension was added to each well separately to a density of 50 µL/well. After uniform mixing, the cells were incubated at 4°C for 1 hour. Washing was performed 3 times with PBS. APC anti-mouse IgG Fc antibody was diluted in PBS + 1% BSA solution (1 µL/well, 100 µl). The cells were resuspended and then incubated at 4°C for 0.5 h. Washing was performed 3 times with PBS. The cells were resuspended in 300 µL of PBS and analyzed by flow cytometry. The optimal mice were selected for hybridoma fusion according to the protein-binding activity and cellular titer.

### 2.2 Hybridoma fusion and screening

Seven days after hybridoma fusion, supernatant screening was performed using ELISA. Specifically, monkey ADAM9 protein was diluted to 1 µg/ml in coating solution, added at 100 µl/well, and then coated overnight at 4°C. Washing was performed once with 300 µL of PBST. 100 µl of PBS + 2% BSA was added to each well and then incubated at 37°C for 1 hour. Then, 20 µl of hybridoma supernatant was directly added to the microplate and then incubated at 37°C for 2 hours. The solution was discarded, and the microplate was washed 3 times with PBST at 320 µl/well. The microplate was drained. 100 µL of HRP-Goat anti-mouse IgG (Thermo Fisher) diluted at a ratio of 1 : 10,000 was added to each well and then incubated at 37°C for 1 hour. The solution was discarded, and the microplate was washed 5 times with PBST. The microplate was drained. 80 µL of TMB (Huzhou InnoReagents Co., Ltd.) was added to each well for color development in the dark. Then, 40 µL of 2N H₂SO₄ was added to terminate the reaction, and the absorbance at 450 nm was read using a microplate reader. Clones with a signal value 5-fold higher than that of the negative control were further validated using ELISA with human ADAM9 protein and flow cytometry with the overexpressing cells prepared in section 1.3. Positive clones were subjected to subcloning. Monoclonal clones were screened using the above method, and the optimal monoclonal clones were selected for scale-up culture.

### Example 3: Evaluation of anti-human ADAM9 murine antibodies

### 3.1 Evaluation of binding activity of anti-human ADAM9 murine antibodies

All selected monoclonal clones were scaled up for serum-free culture. The 5-10 ml culture supernatants were affinity-purified using Protein-A beads. The eluted products were neutralized with Tris solution. The antibody protein concentrations were quantified using a Nanodrop and then used for candidate evaluation.

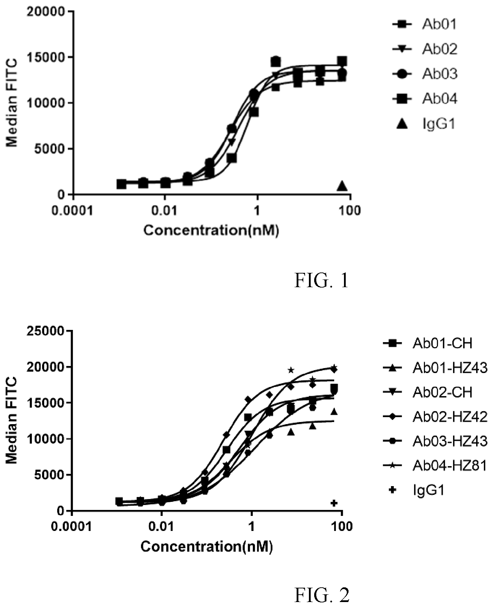

Adherent lung squamous cell carcinoma cells EBC-1 (Nanjing Cobioer) were digested with StemProTM Accutase (Gibco) solution and counted. An appropriate number of cells were taken, washed twice with 1× PBS, and resuspended in 1% BSA solution. Then, the cells were transferred to a 96-well V-bottom plate at 50 µL/well. Candidate antibodies were diluted in 1% BSA, starting at 10 µg/mL with a 3-fold gradient dilution. Then, 50 µL of the diluted antibodies was added to the V-bottom plate containing the cells, and the mixture was incubated at 4°C for 45 min. The cells were washed twice with PBS. Then, 50 µL of diluted secondary antibody was added to each well. After uniform mixing, the mixture was incubated at 4°C for 30 min. The cells were washed twice with PBS. Then, the cells were resuspended in 400 µL of PBS and analyzed by flow cytometry. Data processing: The Median PE values were exported and then imported into GraphPad Prism 6 software to calculate EC₅₀. The results are as shown in FIG. 1 and Table 2. The antibodies Ab01 to Ab04 all exhibited high affinity, at the 10E-10 level.

**Table 2: Determination of affinity of anti-human ADAM9 murine antibodies for EBC-1 cells**

| Abs | EC₅₀ (nM) |
|---|---|
| Ab01 | 0.25 |
| Ab02 | 0.39 |
| Ab03 | 0.28 |
| Ab04 | 0.61 |

### 3.2 Sequencing of anti-human ADAM9 murine antibodies and construction of chimeric antibodies

Hybridoma cells were cultured to around 8000 cells and then lysed. The first-strand cDNA was synthesized using a cDNA reverse transcription kit (Thermo Fisher). VH and VK genes were amplified from cDNA by PCR using primers and then sequenced. The variable region sequences of anti-human ADAM9 antibodies were obtained, as shown in Table 3.

**Table 3. Amino acid sequences of variable regions and CDRs of anti-human ADAM9 murine antibodies**

| Antibody | VH | VL | Numbering system | CDR H1 | CDR H2 | CDR H3 | CDR L1 | CDR L2 | CDR L3 |
|---|---|---|---|---|---|---|---|---|---|
| | SEQ ID NO: | | | SEQ ID NO: | | | | | |
| Ab01 | 1 | 2 | Chothia | 11 | 12 | 13 | 14 | 15 | 16 |
| | | | AbM | 17 | 18 | 13 | 14 | 15 | 16 |
| | | | Kabat | 19 | 20 | 13 | 14 | 15 | 16 |
| | | | IMGT | 21 | 22 | 94 | 24 | 25 | 16 |
| Ab02 | 7 | 8 | Chothia | 95 | 96 | 27 | 97 | 98 | 30 |
| | | | AbM | 99 | 100 | 27 | 97 | 98 | 30 |
| | | | Kabat | 101 | 102 | 27 | 97 | 98 | 30 |
| | | | IMGT | 103 | 104 | 37 | 105 | 39 | 30 |
| Ab03 | 48 | 49 | Chothia | 56 | 57 | 58 | 59 | 60 | 61 |
| | | | AbM | 62 | 63 | 64 | 59 | 60 | 61 |
| | | | Kabat | 62 | 63 | 64 | 59 | 60 | 61 |
| | | | IMGT | 65 | 66 | 67 | 68 | 69 | 70 |
| | | | Chothia | 71 | 72 | 73 | 74 | 75 | 76 |
| Ab04 | 52 | 53 | AbM | 77 | 78 | 73 | 74 | 75 | 76 |
| | | | Kabat | 79 | 80 | 73 | 74 | 75 | 76 |
| | | | IMGT | 81 | 82 | 83 | 84 | 85 | 76 |

### Example 4: Humanization evaluation of anti-human ADAM9 antibodies

### 4.1 Humanization and expression of anti-human ADAM9 antibodies

Humanization of the murine antibodies Ab01, Ab02, Ab03 and Ab04 was performed using the CDR grafting method for antibody humanization. Briefly, humanization involves the following steps: The amino acid sequences of murine monoclonal antibodies were aligned with those of human germline antibodies to identify sequences with high homology and favorable physicochemical properties, which were then used as human germline framework sequences. The HLA-DR affinity was analyzed and investigated to select human germline framework sequences with low affinity. Then, the six CDRs of the murine antibodies were correspondingly grafted onto selected heavy and light chain framework sequences.

Further, computer simulation techniques were employed to analyze the variable regions and their surrounding framework amino acid sequences through molecular docking, to investigate their spatial stereoscopic binding modes. By means of calculating electrostatic forces, van der Waals forces, hydrophilicity-hydrophobicity, and entropy values, key amino acids in the amino acid sequences of the murine antibodies that interact with the ADAM9 protein and maintain the spatial structure were analyzed. These murine-derived amino acids were retained in the grafted antibodies. That is, a series of back mutations was performed on the amino acid residues in the FR regions of the above humanized templates, to allow the humanized antibodies to retain the antigen-binding ability of the murine antibodies as much as possible.

Nanjing GenScript Biotech Co., Ltd. was commissioned to perform codon optimization on the humanized antibodies, and the synthesized cDNA was ligated into the expression plasmid pTT5. The expression plasmids for the heavy and light chains of the humanized antibodies were co-transfected into CHO-S cells. After 7 days of expression, the supernatant was collected by centrifugation. The recombinant antibodies in the supernatant were purified using Protein A (MabSelect SuRe, GE) to obtain anti-human ADAM9 chimeric and humanized antibodies. The sequences of variable regions and CDRs of the humanized antibodies are as shown in Table 4.

**Table 4. Amino acid sequences of variable regions and CDRs of anti-human ADAM9 humanized antibodies**

| Antibody | VH | VL | Numbering system | CDR H1 | CDR H2 | CDR H3 | CDR L1 | CDR L2 | CDR L3 |
|---|---|---|---|---|---|---|---|---|---|
| | SEQ ID NO: | | | SEQ ID NO: | | | | | |
| Ab01-HZ43 | 3 | 4 | Chothia | 11 | 12 | 13 | 14 | 15 | 16 |
| | | | AbM | 17 | 18 | 13 | 14 | 15 | 16 |
| | | | Kabat | 19 | 20 | 13 | 14 | 15 | 16 |
| | | | IMGT | 21 | 22 | 23 | 24 | 25 | 16 |
| Ab02-HZ42 | 9 | 10 | Chothia | 11 | 26 | 27 | 28 | 29 | 30 |
| | | | AbM | 31 | 32 | 27 | 28 | 29 | 30 |
| | | | Kabat | 33 | 34 | 27 | 28 | 29 | 30 |
| | | | IMGT | 35 | 36 | 37 | 38 | 39 | 30 |
| Ab03-HZ43 | 50 | 51 | Chothia | 56 | 57 | 58 | 59 | 60 | 61 |
| | | | AbM | 62 | 63 | 64 | 59 | 60 | 61 |
| | | | Kabat | 62 | 63 | 64 | 59 | 60 | 61 |
| | | | IMGT | 65 | 66 | 67 | 68 | 69 | 70 |
| Ab04-HZ81 | 54 | 55 | Chothia | 71 | 72 | 73 | 74 | 75 | 76 |
| | | | AbM | 77 | 78 | 73 | 74 | 75 | 76 |
| | | | Kabat | 79 | 80 | 73 | 74 | 75 | 76 |
| | | | IMGT | 81 | 82 | 83 | 84 | 85 | 76 |

### 4.2 Protein affinity detection of anti-human ADAM9 humanized antibodies

The dynamic affinities of the chimeric antibodies Ab01-CH and Ab02-CH and the humanized antibodies Ab01-HZ43, Ab02-HZ42, Ab03-HZ43 and Ab04-HZ81 for human and monkey ADAM9-His were detected using ForteBio (Pall Life Sciences).

The specific method was as follows: The test antibodies were diluted to 5 µg/ml in PBST (0.02% Tween 20), and human and monkey ADAM9-His proteins were serially diluted to 200 nM, 100 nM, 50 nM, 25 nM, 12.50 nM, 6.25 nM, 3.125 nM and 0 nM. Subsequently, the test antibodies were captured for 60 s using Protein A Senso (Pall Life Sciences) in PBST (0.02% Tween-20) solution, followed by association with human or monkey ADAM9-His proteins for 60 s and dissociation for 180 s. The results were obtained. The obtained results were imported into Data Analysis 11.0 software. The 1 : 1 model with global fitting in the software was selected to analyze the results, so as to obtain the association rate, dissociation rate and affinity constant.

The results are as shown in Table 5. The dissociation rate constants (kdis) of Ab04-HZ81, Ab01-HZ43 and Ab02-HZ42 for human ADAM9-His were lower than that of the positive control hMAB-A (2I .2), indicating high dynamic affinities. The dissociation constant and dissociation rate constant of Ab04-HZ81 for monkey ADAM9-His reached the same level as those of the positive control hMAB-A (2I .2), indicating high dynamic affinities.

**Table 5 Dynamic affinity detection of anti-human ADAM9 humanized antibodies for human and monkey ADAM9-His**

| Dynamic affinity detection of anti-human ADAM9 humanized antibodies for human ADAM9-His | | | |
|---|---|---|---|
| Abs | KD (M) | kon (1/Ms) | kdis (1/s) |
| hMAB-A(2I .2) | 1.23E-09 | 6.96E+05 | 8.59E-04 |
| Ab01-CH | 4.43E-09 | 1.87E+05 | 8.29E-04 |
| Ab01-HZ43 | 4.03E-09 | 1.75E+05 | 7.03E-04 |
| Ab02-CH | 2.00E-09 | 7.84E+05 | 1.57E-03 |
| Ab02-HZ42 | 3.99E-09 | 5.62E+05 | 2.24E-03 |
| Ab03-HZ43 | 5.80E-09 | 1.26E+05 | 7.29E-04 |
| Ab04-HZ81 | 2.28E-09 | 9.47E+04 | 2.16E-04 |

| Dynamic affinity detection of anti-human ADAM9 humanized antibodies for monkey ADAM9-His | | | |
|---|---|---|---|
| Abs | KD (M) | kon (1/Ms) | kdis (1/s) |
| hMAB-A(2I .2) | <1.0E-12 | 2.37E+05 | <1.0E-07 |
| Ab01-CH | 1.18E-08 | 8.89E+04 | 1.05E-03 |
| Ab01-HZ43 | 1.19E-08 | 9.28E+04 | 1.10E-03 |
| Ab02-CH | 1.05E-09 | 2.85E+05 | 2.99E-04 |
| Ab02-HZ42 | 3.40E-09 | 1.84E+05 | 6.27E-04 |
| Ab03-HZ43 | 2.92E-09 | 4.16E+04 | 1.21E-04 |
| Ab04-HZ81 | <1.0E-12 | 3.02E+04 | <1.0E-07 |

### 4.3 Cell affinity detection of anti-human ADAM9 humanized antibodies

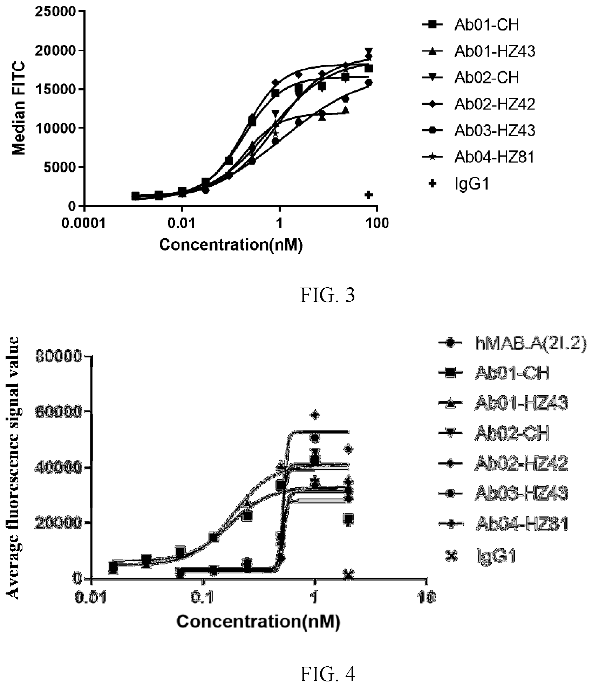

Adherent lung squamous cell carcinoma cells EBC-1 (Nanjing Cobioer) and human breast cancer cells HCC1806 (Nanjing Cobioer) were digested with StemProTM Accutase (Gibco) solution. After counting, an appropriate number of cells were taken, washed twice with 1× PBS, and resuspended in 1% BSA solution. Then, the cells were transferred to a 96-well V-bottom plate at 50 µL/well. Candidate antibodies were diluted in 1% BSA, starting at 10 µg/mL with a 3-fold gradient dilution. Then, 50 µL of the diluted antibodies was added to the V-bottom plate containing the cells, and the mixture was incubated at 4°C for 45 min. The cells were washed twice with PBS. Then, 50 µL of diluted secondary antibody was added to each well. After uniform mixing, the mixture was incubated at 4°C for 30 min. The cells were washed twice with PBS. Then, the cells were resuspended in 400 µL of PBS and analyzed by flow cytometry. Data processing: The Median PE values were exported and then imported into GraphPad Prism 6 software to calculate EC₅₀. The results are as shown in FIG. 2, FIG. 3 and Table 6. The antibodies Ab01-CH, Ab01-HZ43, Ab02-CH, Ab02-HZ42, Ab03-HZ43 and Ab04-HZ81 all exhibited high affinity.

**Table 6 Cell affinity detection of anti-human ADAM9 humanized antibodies**

| Abs | EBC-1 (nM) | HCC1806 (nM) |
|---|---|---|
| Ab01-CH | 0.28 | 0.19 |
| Ab01-HZ43 | 0.33 | 0.19 |
| Ab02-CH | 0.54 | 0.63 |
| Ab02-HZ42 | 0.23 | 0.19 |
| Ab03-HZ43 | 1.20 | 1.22 |
| Ab04-HZ81 | 1.02 | 0.87 |

### 4.4 Endocytic activity detection of anti-human ADAM9 humanized antibodies

The endocytic activity of the humanized anti-human ADAM9 antibody in human lung squamous cell carcinoma EBC-1 cells (Nanjing Cobioer) was detected using a flow cytometer (Thermo, model: Attune NxT). The specific procedures were as follows: EBC-1 cells were plated at 10,000 cells/well and incubated overnight. The test antibodies were diluted in culture medium, starting at 0.3 µg/ml with a 2-fold serial dilution. PHrodo was diluted to 12 µg/ml in culture medium. The serially diluted antibodies were uniformly mixed with PHrodo at a ratio of 1 : 1, and then the mixture was incubated at room temperature for 30 min in the dark. Then, 50 µL of fresh complete medium and 50 µL of the labeled antibodies were added to the cells, followed by co-incubation at 37°C with 5% CO₂ for 24 h. The cells were washed with PBS and analyzed by flow cytometry. Data processing: The fluorescence signal values were exported and then imported into GraphPad Prism 6 software to calculate EC₅₀. The results are as shown in FIG. 4 and Table 7. The chimeric antibody Ab01-CH and the corresponding humanized antibody Ab01-HZ43 exhibited the strongest endocytic activity, while Ab02-HZ42, Ab03-HZ43 and Ab04-HZ81 exhibited endocytic activity similar to the control antibody hMAB-A(2I .2).

**Table 7 Endocytic activity detection of anti-human ADAM9 humanized antibodies**

| Abs | EBC-1 (nM) |
|---|---|
| hMAB-A(2I .2) | 0.52 |
| Ab01-CH | 0.18 |
| Ab01-HZ43 | 0.19 |
| Ab02-CH | 0.52 |
| Ab02-HZ42 | 0.53 |
| Ab03-HZ43 | 0.54 |
| Ab04-HZ81 | 0.51 |

### 4.5 Hydrophobicity detection of anti-human ADAM9 humanized antibodies

Hydrophobicity was detected using hydrophobic interaction chromatography, with conditions as follows: chromatographic column: TOSOH TSKgel Butyl-NPR 100*4.6 mm, 2.5 µm; mobile phase A: 25 mmol/L disodium hydrogen phosphate, pH 7.0, 25% isopropanol; mobile phase B: 1.5 mol/L ammonium sulfate. Samples were diluted to 1 mg/ml with 0.75 mol/L ammonium sulfate. 40 µg of each sample was injected. Gradient elution was performed, where hydrophilic samples were eluted first and hydrophobic samples were eluted later. The hydrophilicity or hydrophobicity of the samples was determined according to their retention times. The results are as shown in Table 8. Ab01-HZ43, Ab02-HZ42, Ab03-HZ43 and Ab04-HZ81 all exhibited good hydrophilicity which was superior to that of the control antibody hMAB-A (2I .2).

**Table 8 Hydrophilicity detection of anti-human ADAM9 humanized antibodies**

| Antibody | Main peak retention time (min) |
|---|---|
| hMAB-A(2I .2) | 19.668 |
| Ab01-HZ43 | 11.089 |
| Ab02-HZ42 | 15.721 |
| Ab03-HZ43 | 15.441 |
| Ab04-HZ81 | 15.929 |

### 4.6 Specificity detection of humanized anti-human ADAM9 antibodies

To detect the specificity of humanized anti-human ADAM9 antibodies, human ADAM9, ADAM12 (Sino Biological) and ADAM15 (Sino Biological) proteins were diluted to 1 µg/ml in CBS coating solution. 100 µL/well of dilution was coated onto a 96-well microplate and incubated at 4°C overnight. The microplate was washed once with PBS and drained. Then, 100 µL of 2% BSA was added to each well and then incubated at 37°C for 2 hours. After that, the blocking solution was removed. The test antibodies were diluted to 10 µg/ml in 2% BSA. Then, 100 µL of the diluted antibodies was added to each well of the microplate and then incubated at 37°C for 2 hours. The plate was washed 3 times with PBST (containing 0.2% Tween-20) and drained. Then, 100 µL of HRP Goat Anti-Human IgG (H+L) (Jackson) was added to each well and then incubated at 37°C for 1 hour. The plate was washed 5 times with PBST (0.2% Tween-20) and drained. Then, 100 µL of TMB chromogenic substrate (Huzhou InnoReagents) was added to each well. After reaction at room temperature for 3-5 min, 50 µL of 2N H₂SO₄ was added to each well to terminate the chromogenic reaction, and the absorbance at OD450 nm was read using a microplate reader.

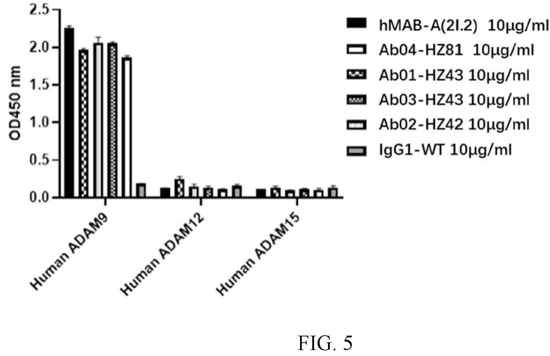

The results are as shown in FIG. 5. All the tested ADAM9 antibodies bound only to the ADAM9 protein, and did not bind to the ADAM12 or ADAM15 proteins.

Although the specific embodiments of the present disclosure have been described in detail, it will be understood by those skilled in the art that various modifications and changes in the details can be made according to all the teachings disclosed and these changes are within the scope of the protection of the present disclosure. The full scope of the present disclosure is given by the appended claims and any equivalents thereof.

## Claims

1. An antibody or an antigen-binding fragment thereof specifically binding to ADAM9, wherein the antibody or the antigen-binding fragment thereof comprises complementarity determining regions (CDRs) as follows:
(a) CDR-H1, CDR-H2 and CDR-H3 contained in a heavy chain variable region (VH) set forth in SEQ ID NO: 54 or 52; and/or, CDR-L1, CDR-L2 and CDR-L3 contained in a light chain variable region (VL) set forth in SEQ ID NO: 55 or 53;
(b) CDR-H1, CDR-H2 and CDR-H3 contained in a heavy chain variable region (VH) set forth in SEQ ID NO: 3 or 1; and/or, CDR-L1, CDR-L2 and CDR-L3 contained in a light chain variable region (VL) set forth in SEQ ID NO: 4 or 2;
(c) CDR-H1, CDR-H2 and CDR-H3 contained in a heavy chain variable region (VH) set forth in SEQ ID NO: 9 or 7; and/or, CDR-L1, CDR-L2 and CDR-L3 contained in a light chain variable region (VL) set forth in SEQ ID NO: 10 or 8;
(d) CDR-H1, CDR-H2 and CDR-H3 contained in a heavy chain variable region (VH) set forth in SEQ ID NO: 50 or 48; and/or, CDR-L1, CDR-L2 and CDR-L3 contained in a light chain variable region (VL) set forth in SEQ ID NO: 51 or 49; or
(e) CDR-H1, CDR-H2 and CDR-H3 contained in a heavy chain variable region (VH) described below, and/or CDR-L1, CDR-L2 and CDR-L3 contained in a light chain variable region (VL) described below, wherein the heavy chain variable region (VH) and/or the light chain variable region (VL) comprises a mutation in at least one CDR compared to the heavy chain variable region and/or light chain variable region of any one of (a) to (d), and the mutation is a substitution, deletion or addition of one or several amino acids (e.g., a substitution, deletion or addition of 1, 2 or 3 amino acids); preferably, the substitution is a conservative substitution;
preferably, the CDRs are defined according to the IMGT, Kabat, Chothia or AbM numbering system.

2. The antibody or the antigen-binding fragment thereof according to claim 1, wherein the antibody or the antigen-binding fragment thereof comprises:
(1) a heavy chain variable region (VH) and/or a light chain variable region (VL) described below, wherein CDRs are defined by the IMGT numbering system:
(1a) a heavy chain variable region (VH) comprising 3 CDRs as follows: CDR-H1 having a sequence of SEQ ID NO: 81 or a variant thereof; CDR-H2 having a sequence of SEQ ID NO: 82 or a variant thereof; and CDR-H3 having a sequence of SEQ ID NO: 83 or a variant thereof; and/or, a light chain variable region (VL) comprising 3 CDRs as follows: CDR-L1 having a sequence of SEQ ID NO: 84 or a variant thereof; CDR-L2 having a sequence of SEQ ID NO: 85 or a variant thereof; and CDR-L3 having a sequence of SEQ ID NO: 76 or a variant thereof;
(1b-i) a heavy chain variable region (VH) comprising 3 CDRs as follows: CDR-H1 having a sequence of SEQ ID NO: 21 or a variant thereof; CDR-H2 having a sequence of SEQ ID NO: 22 or a variant thereof; and CDR-H3 having a sequence of SEQ ID NO: 23 or a variant thereof; and/or, a light chain variable region (VL) comprising 3 CDRs as follows: CDR-L1 having a sequence of SEQ ID NO: 24 or a variant thereof; CDR-L2 having a sequence of SEQ ID NO: 25 or a variant thereof; and CDR-L3 having a sequence of SEQ ID NO: 16 or a variant thereof;
(1b-ii) a heavy chain variable region (VH) comprising 3 CDRs as follows: CDR-H1 having a sequence of SEQ ID NO: 21 or a variant thereof; CDR-H2 having a sequence of SEQ ID NO: 22 or a variant thereof; and CDR-H3 having a sequence of SEQ ID NO: 94 or a variant thereof; and/or, a light chain variable region (VL) comprising 3 CDRs as follows: CDR-L1 having a sequence of SEQ ID NO: 24 or a variant thereof; CDR-L2 having a sequence of SEQ ID NO: 25 or a variant thereof; and CDR-L3 having a sequence of SEQ ID NO: 16 or a variant thereof;
(1c-i) a heavy chain variable region (VH) comprising 3 CDRs as follows: CDR-H1 having a sequence of SEQ ID NO: 35 or a variant thereof; CDR-H2 having a sequence of SEQ ID NO: 36 or a variant thereof; and CDR-H3 having a sequence of SEQ ID NO: 37 or a variant thereof; and/or, a light chain variable region (VL) comprising 3 CDRs as follows: CDR-L1 having a sequence of SEQ ID NO: 38 or a variant thereof; CDR-L2 having a sequence of SEQ ID NO: 39 or a variant thereof; and CDR-L3 having a sequence of SEQ ID NO: 30 or a variant thereof;
(1c-ii) a heavy chain variable region (VH) comprising 3 CDRs as follows: CDR-H1 having a sequence of SEQ ID NO: 103 or a variant thereof; CDR-H2 having a sequence of SEQ ID NO: 104 or a variant thereof; and CDR-H3 having a sequence of SEQ ID NO: 37 or a variant thereof; and/or, a light chain variable region (VL) comprising 3 CDRs as follows: CDR-L1 having a sequence of SEQ ID NO: 105 or a variant thereof; CDR-L2 having a sequence of SEQ ID NO: 39 or a variant thereof; and CDR-L3 having a sequence of SEQ ID NO: 30 or a variant thereof; or
(1d) a heavy chain variable region (VH) comprising 3 CDRs as follows: CDR-H1 having a sequence of SEQ ID NO: 65 or a variant thereof; CDR-H2 having a sequence of SEQ ID NO: 66 or a variant thereof; and CDR-H3 having a sequence of SEQ ID NO: 67 or a variant thereof; and/or, a light chain variable region (VL) comprising 3 CDRs as follows: CDR-L1 having a sequence of SEQ ID NO: 68 or a variant thereof; CDR-L2 having a sequence of SEQ ID NO: 69 or a variant thereof; and CDR-L3 having a sequence of SEQ ID NO: 70 or a variant thereof;
or,
(2) a heavy chain variable region (VH) and/or a light chain variable region (VL) described below, wherein CDRs are defined by the Chothia numbering system:
(2a) a heavy chain variable region (VH) comprising 3 CDRs as follows: CDR-H1 having a sequence of SEQ ID NO: 71 or a variant thereof; CDR-H2 having a sequence of SEQ ID NO: 72 or a variant thereof; and CDR-H3 having a sequence of SEQ ID NO: 73 or a variant thereof; and/or, a light chain variable region (VL) comprising 3 CDRs as follows: CDR-L1 having a sequence of SEQ ID NO: 74 or a variant thereof; CDR-L2 having a sequence of SEQ ID NO: 75 or a variant thereof; and CDR-L3 having a sequence of SEQ ID NO: 76 or a variant thereof;
(2b) a heavy chain variable region (VH) comprising 3 CDRs as follows: CDR-H1 having a sequence of SEQ ID NO: 11 or a variant thereof; CDR-H2 having a sequence of SEQ ID NO: 12 or a variant thereof; and CDR-H3 having a sequence of SEQ ID NO: 13 or a variant thereof; and/or, a light chain variable region (VL) comprising 3 CDRs as follows: CDR-L1 having a sequence of SEQ ID NO: 14 or a variant thereof; CDR-L2 having a sequence of SEQ ID NO: 15 or a variant thereof; and CDR-L3 having a sequence of SEQ ID NO: 16 or a variant thereof;
(2c-i) a heavy chain variable region (VH) comprising 3 CDRs as follows: CDR-H1 having a sequence of SEQ ID NO: 11 or a variant thereof; CDR-H2 having a sequence of SEQ ID NO: 26 or a variant thereof; and CDR-H3 having a sequence of SEQ ID NO: 27 or a variant thereof; and/or, a light chain variable region (VL) comprising 3 CDRs as follows: CDR-L1 having a sequence of SEQ ID NO: 28 or a variant thereof; CDR-L2 having a sequence of SEQ ID NO: 29 or a variant thereof; and CDR-L3 having a sequence of SEQ ID NO: 30 or a variant thereof;
(2c-ii) a heavy chain variable region (VH) comprising 3 CDRs as follows: CDR-H1 having a sequence of SEQ ID NO: 95 or a variant thereof; CDR-H2 having a sequence of SEQ ID NO: 96 or a variant thereof; and CDR-H3 having a sequence of SEQ ID NO: 27 or a variant thereof; and/or, a light chain variable region (VL) comprising 3 CDRs as follows: CDR-L1 having a sequence of SEQ ID NO: 97 or a variant thereof; CDR-L2 having a sequence of SEQ ID NO: 98 or a variant thereof; and CDR-L3 having a sequence of SEQ ID NO: 30 or a variant thereof; or
(2d) a heavy chain variable region (VH) comprising 3 CDRs as follows: CDR-H1 having a sequence of SEQ ID NO: 56 or a variant thereof; CDR-H2 having a sequence of SEQ ID NO: 57 or a variant thereof; and CDR-H3 having a sequence of SEQ ID NO: 58 or a variant thereof; and/or, a light chain variable region (VL) comprising 3 CDRs as follows: CDR-L1 having a sequence of SEQ ID NO: 59 or a variant thereof; CDR-L2 having a sequence of SEQ ID NO: 60 or a variant thereof; and CDR-L3 having a sequence of SEQ ID NO: 61 or a variant thereof;
or,
(3) a heavy chain variable region (VH) and/or a light chain variable region (VL) described below, wherein CDRs are defined by the Kabat numbering system:
(3a) a heavy chain variable region (VH) comprising 3 CDRs as follows: CDR-H1 having a sequence of SEQ ID NO: 79 or a variant thereof; CDR-H2 having a sequence of SEQ ID NO: 80 or a variant thereof; and CDR-H3 having a sequence of SEQ ID NO: 73 or a variant thereof; and/or, a light chain variable region (VL) comprising 3 CDRs as follows: CDR-L1 having a sequence of SEQ ID NO: 74 or a variant thereof; CDR-L2 having a sequence of SEQ ID NO: 75 or a variant thereof; and CDR-L3 having a sequence of SEQ ID NO: 76 or a variant thereof;
(3b) a heavy chain variable region (VH) comprising 3 CDRs as follows: CDR-H1 having a sequence of SEQ ID NO: 19 or a variant thereof; CDR-H2 having a sequence of SEQ ID NO: 20 or a variant thereof; and CDR-H3 having a sequence of SEQ ID NO: 13 or a variant thereof; and/or, a light chain variable region (VL) comprising 3 CDRs as follows: CDR-L1 having a sequence of SEQ ID NO: 14 or a variant thereof; CDR-L2 having a sequence of SEQ ID NO: 15 or a variant thereof; and CDR-L3 having a sequence of SEQ ID NO: 16 or a variant thereof;
(3c-i) a heavy chain variable region (VH) comprising 3 CDRs as follows: CDR-H1 having a sequence of SEQ ID NO: 33 or a variant thereof; CDR-H2 having a sequence of SEQ ID NO: 34 or a variant thereof; and CDR-H3 having a sequence of SEQ ID NO: 27 or a variant thereof; and/or, a light chain variable region (VL) comprising 3 CDRs as follows: CDR-L1 having a sequence of SEQ ID NO: 28 or a variant thereof; CDR-L2 having a sequence of SEQ ID NO: 29 or a variant thereof; and CDR-L3 having a sequence of SEQ ID NO: 30 or a variant thereof;
(3c-ii) a heavy chain variable region (VH) comprising 3 CDRs as follows: CDR-H1 having a sequence of SEQ ID NO: 101 or a variant thereof; CDR-H2 having a sequence of SEQ ID NO: 102 or a variant thereof; and CDR-H3 having a sequence of SEQ ID NO: 27 or a variant thereof; and/or, a light chain variable region (VL) comprising 3 CDRs as follows: CDR-L1 having a sequence of SEQ ID NO: 97 or a variant thereof; CDR-L2 having a sequence of SEQ ID NO: 98 or a variant thereof; and CDR-L3 having a sequence of SEQ ID NO: 30 or a variant thereof; or
(3d) a heavy chain variable region (VH) comprising 3 CDRs as follows: CDR-H1 having a sequence of SEQ ID NO: 62 or a variant thereof; CDR-H2 having a sequence of SEQ ID NO: 63 or a variant thereof; and CDR-H3 having a sequence of SEQ ID NO: 64 or a variant thereof; and/or, a light chain variable region (VL) comprising 3 CDRs as follows: CDR-L1 having a sequence of SEQ ID NO: 59 or a variant thereof; CDR-L2 having a sequence of SEQ ID NO: 60 or a variant thereof; and CDR-L3 having a sequence of SEQ ID NO: 61 or a variant thereof;
or,
(4) a heavy chain variable region (VH) and/or a light chain variable region (VL) described below, wherein CDRs are defined by the AbM numbering system:
(4a) a heavy chain variable region (VH) comprising 3 CDRs as follows: CDR-H1 having a sequence of SEQ ID NO: 77 or a variant thereof; CDR-H2 having a sequence of SEQ ID NO: 78 or a variant thereof; and CDR-H3 having a sequence of SEQ ID NO: 73 or a variant thereof; and/or, a light chain variable region (VL) comprising 3 CDRs as follows: CDR-L1 having a sequence of SEQ ID NO: 74 or a variant thereof; CDR-L2 having a sequence of SEQ ID NO: 75 or a variant thereof; and CDR-L3 having a sequence of SEQ ID NO: 76 or a variant thereof;
(4b) a heavy chain variable region (VH) comprising 3 CDRs as follows: CDR-H1 having a sequence of SEQ ID NO: 17 or a variant thereof; CDR-H2 having a sequence of SEQ ID NO: 18 or a variant thereof; and CDR-H3 having a sequence of SEQ ID NO: 13 or a variant thereof; and/or, a light chain variable region (VL) comprising 3 CDRs as follows: CDR-L1 having a sequence of SEQ ID NO: 14 or a variant thereof; CDR-L2 having a sequence of SEQ ID NO: 15 or a variant thereof; and CDR-L3 having a sequence of SEQ ID NO: 16 or a variant thereof;
(4c-i) a heavy chain variable region (VH) comprising 3 CDRs as follows: CDR-H1 having a sequence of SEQ ID NO: 31 or a variant thereof; CDR-H2 having a sequence of SEQ ID NO: 32 or a variant thereof; and CDR-H3 having a sequence of SEQ ID NO: 27 or a variant thereof; and/or, a light chain variable region (VL) comprising 3 CDRs as follows: CDR-L1 having a sequence of SEQ ID NO: 28 or a variant thereof; CDR-L2 having a sequence of SEQ ID NO: 29 or a variant thereof; and CDR-L3 having a sequence of SEQ ID NO: 30 or a variant thereof;
(4c-ii) a heavy chain variable region (VH) comprising 3 CDRs as follows: CDR-H1 having a sequence of SEQ ID NO: 99 or a variant thereof; CDR-H2 having a sequence of SEQ ID NO: 100 or a variant thereof; and CDR-H3 having a sequence of SEQ ID NO: 27 or a variant thereof; and/or, a light chain variable region (VL) comprising 3 CDRs as follows: CDR-L1 having a sequence of SEQ ID NO: 97 or a variant thereof; CDR-L2 having a sequence of SEQ ID NO: 98 or a variant thereof; and CDR-L3 having a sequence of SEQ ID NO: 30 or a variant thereof; or
(4d) a heavy chain variable region (VH) comprising 3 CDRs as follows: CDR-H1 having a sequence of SEQ ID NO: 62 or a variant thereof; CDR-H2 having a sequence of SEQ ID NO: 63 or a variant thereof; and CDR-H3 having a sequence of SEQ ID NO: 64 or a variant thereof; and/or, a light chain variable region (VL) comprising 3 CDRs as follows: CDR-L1 having a sequence of SEQ ID NO: 59 or a variant thereof; CDR-L2 having a sequence of SEQ ID NO: 60 or a variant thereof; and CDR-L3 having a sequence of SEQ ID NO: 61 or a variant thereof;
wherein the variant in any one of (1a), (1b-i), (1b-ii), (1c-i), (1c-ii), (1d), (2a), (2b), (2c-i), (2c-ii), (2d), (3a), (3b), (3c-i), (3c-ii), (3d), (4a), (4b), (4c-i), (4c-ii) and (4d) has a substitution, deletion or addition of one or several amino acids (e.g., a substitution, deletion or addition of 1, 2 or 3 amino acids) compared to the sequence from which the variant is derived; preferably, the substitution is a conservative substitution.

3. The antibody or the antigen-binding fragment thereof according to claim 1 or 2, wherein the antibody or the antigen-binding fragment thereof comprises:
(a-i) VH comprising a sequence as set forth in SEQ ID NO: 54 or a variant thereof and/or VL comprising a sequence as set forth in SEQ ID NO: 55 or a variant thereof;
(a-ii) VH comprising a sequence as set forth in SEQ ID NO: 52 or a variant thereof and/or VL comprising a sequence as set forth in SEQ ID NO: 53 or a variant thereof;
(b-i) VH comprising a sequence as set forth in SEQ ID NO: 3 or a variant thereof and/or VL comprising a sequence as set forth in SEQ ID NO: 4 or a variant thereof;
(b-ii) VH comprising a sequence as set forth in SEQ ID NO: 1 or a variant thereof and/or VL comprising a sequence as set forth in SEQ ID NO: 2 or a variant thereof;
(c-i) VH comprising a sequence as set forth in SEQ ID NO: 9 or a variant thereof and/or VL comprising a sequence as set forth in SEQ ID NO: 10 or a variant thereof;
(c-ii) VH comprising a sequence as set forth in SEQ ID NO: 7 or a variant thereof and/or VL comprising a sequence as set forth in SEQ ID NO: 8 or a variant thereof;
(d-i) VH comprising a sequence as set forth in SEQ ID NO: 50 or a variant thereof and/or VL comprising a sequence as set forth in SEQ ID NO: 51 or a variant thereof; or
(d-ii) VH comprising a sequence as set forth in SEQ ID NO: 48 or a variant thereof and/or VL comprising a sequence as set forth in SEQ ID NO: 49 or a variant thereof;
wherein the variant has at least 80%, at least 85%, at least 90%, at least 91%, at least 92%, at least 93%, at least 94%, at least 95%, at least 96%, at least 97%, at least 98%, at least 99% or 100% sequence identity to the sequence from which the variant is derived, or has a substitution, deletion or addition of one or several amino acids (e.g., a substitution, deletion or addition of 1, 2, 3, 4 or 5 amino acids) compared to the sequence from which the variant is derived; preferably, the substitution is a conservative substitution.

4. The antibody or the antigen-binding fragment thereof according to any one of claims 1 to 3, wherein the antibody or the antigen-binding fragment thereof is a murine antibody, a chimeric antibody or a humanized antibody.

5. The antibody or the antigen-binding fragment thereof according to any one of claims 1 to 4, wherein the antibody or the antigen-binding fragment thereof further comprises a constant region from or derived from a human immunoglobulin;
preferably, the heavy chain of the antibody or the antigen-binding fragment thereof comprises a heavy chain constant region from or derived from a human immunoglobulin (e.g., IgG1, IgG2, IgG3 or IgG4); preferably, the antibody or the antigen-binding fragment thereof comprises a wild-type Fc region, or a mutated or chemically modified Fc region that has an altered effector function compared to the wild-type Fc region;
preferably, the antibody or the antigen-binding fragment thereof comprises a variant of the human IgG1 heavy chain constant region, wherein the variant has the following substitutions compared to the wild-type sequence from which the variant is derived: Leu234Ala, Leu235Ala and Gly237Ala (positions according to the EU numbering system);
preferably, the light chain of the antibody or the antigen-binding fragment thereof comprises a light chain constant region from or derived from a human immunoglobulin (e.g., κ or λ);
preferably, the antibody or the antigen-binding fragment thereof comprises a heavy chain constant region (CH) as set forth in SEQ ID NO: 5 or a variant thereof, wherein the variant has a conservative substitution of up to 20 amino acids (e.g., a conservative substitution of up to 15, up to 10, or up to 5 amino acids; e.g., a conservative substitution of 1, 2, 3, 4 or 5 amino acids) compared to SEQ ID NO: 5;
preferably, the antibody or the antigen-binding fragment thereof comprises a light chain constant region (CL) as set forth in SEQ ID NO: 6 or a variant thereof, wherein the variant has a conservative substitution of up to 20 amino acids (e.g., a conservative substitution of up to 15, up to 10, or up to 5 amino acids; e.g., a conservative substitution of 1, 2, 3, 4 or 5 amino acids) compared to SEQ ID NO: 6;
more preferably, the antibody or the antigen-binding fragment thereof comprises a heavy chain constant region (CH) as set forth in SEQ ID NO: 5 and a light chain constant region (CL) as set forth in SEQ ID NO: 6.

6. The antibody or the antigen-binding fragment thereof according to claim 5, wherein the heavy chain constant region (CH) as set forth in SEQ ID NO: 5 or the variant thereof lacks a C-terminal lysine.

7. The antibody or the antigen-binding fragment thereof according to any one of claims 1 to 6, wherein the antibody or the antigen-binding fragment thereof comprises:
(1) a heavy chain comprising VH set forth in SEQ ID NO: 54 and a heavy chain constant region (CH) set forth in SEQ ID NO: 5; and a light chain comprising VL set forth in SEQ ID NO: 55 and a light chain constant region (CL) set forth in SEQ ID NO: 6;
(2) a heavy chain comprising VH set forth in SEQ ID NO: 52 and a heavy chain constant region (CH) set forth in SEQ ID NO: 5; and a light chain comprising VL set forth in SEQ ID NO: 53 and a light chain constant region (CL) set forth in SEQ ID NO: 6;
(3) a heavy chain comprising VH set forth in SEQ ID NO: 3 and a heavy chain constant region (CH) set forth in SEQ ID NO: 5; and a light chain comprising VL set forth in SEQ ID NO: 4 and a light chain constant region (CL) set forth in SEQ ID NO: 6;
(4) a heavy chain comprising VH set forth in SEQ ID NO: 1 and a heavy chain constant region (CH) set forth in SEQ ID NO: 5; and a light chain comprising VL set forth in SEQ ID NO: 2 and a light chain constant region (CL) set forth in SEQ ID NO: 6;
(5) a heavy chain comprising VH set forth in SEQ ID NO: 9 and a heavy chain constant region (CH) set forth in SEQ ID NO: 5; and a light chain comprising VL set forth in SEQ ID NO: 10 and a light chain constant region (CL) set forth in SEQ ID NO: 6;
(6) a heavy chain comprising VH set forth in SEQ ID NO: 7 and a heavy chain constant region (CH) set forth in SEQ ID NO: 5; and a light chain comprising VL set forth in SEQ ID NO: 8 and a light chain constant region (CL) set forth in SEQ ID NO: 6;
(7) a heavy chain comprising VH set forth in SEQ ID NO: 50 and a heavy chain constant region (CH) set forth in SEQ ID NO: 5; and a light chain comprising VL set forth in SEQ ID NO: 51 and a light chain constant region (CL) set forth in SEQ ID NO: 6; or
(8) a heavy chain comprising VH set forth in SEQ ID NO: 48 and a heavy chain constant region (CH) set forth in SEQ ID NO: 5; and a light chain comprising VL set forth in SEQ ID NO: 49 and a light chain constant region (CL) set forth in SEQ ID NO: 6.

8. The antibody or the antigen-binding fragment thereof according to claim 3 or 7, wherein the N-terminal glutamine of the VH comprising a sequence as set forth in SEQ ID NO: 54, SEQ ID NO: 52, SEQ ID NO: 3, SEQ ID NO: 1, SEQ ID NO: 9, SEQ ID NO: 7, SEQ ID NO: 50 or SEQ ID NO: 48 or a variant thereof undergoes cyclization to form pyroglutamic acid or pyroglutamate.

9. The antibody or the antigen-binding fragment thereof according to any one of claims 1 to 8, wherein the antibody or the antigen-binding fragment thereof comprises:
(1) a heavy chain having a sequence as set forth in SEQ ID NO: 88 and a light chain having a sequence as set forth in SEQ ID NO: 89;
(2) a heavy chain having a sequence as set forth in SEQ ID NO: 40 and a light chain having a sequence as set forth in SEQ ID NO: 41;
(3) a heavy chain having a sequence as set forth in SEQ ID NO: 42 and a light chain having a sequence as set forth in SEQ ID NO: 43; or,
(4) a heavy chain having a sequence as set forth in SEQ ID NO: 86 and a light chain having a sequence as set forth in SEQ ID NO: 87.

10. The antibody or the antigen-binding fragment thereof according to claim 9, wherein the N-terminal glutamine of the heavy chain having a sequence as set forth in SEQ ID NO: 88, SEQ ID NO: 40, SEQ ID NO: 42 or SEQ ID NO: 86 or a variant thereof undergoes cyclization to form pyroglutamic acid or pyroglutamate.

11. The antibody or the antigen-binding fragment thereof according to any one of claims 1 to 10, wherein the antibody or the antigen-binding fragment thereof is selected from ScFv, Fab, Fab', Fab'-SH, F(ab')2, an Fv fragment, disulfide-linked Fv (dsFv), a diabody, a bispecific antibody and a multispecific antibody.

12. The antibody or the antigen-binding fragment thereof according to any one of claims 1 to 11, wherein the antibody or the antigen-binding fragment thereof carries a label; preferably, the antibody or the antigen-binding fragment thereof carries a detectable label, e.g., an enzyme (e.g., horseradish peroxidase), a radionuclide, a fluorescent dye, a luminescent substance (e.g., a chemiluminescent substance) or a biotin.

13. The antibody or the antigen-binding fragment thereof according to any one of claims 1 to 12, wherein the antibody or the antigen-binding fragment thereof has one or more characteristics selected from:
(1) binding to ADAM9 (e.g., human or monkey ADAM9) with an EC₅₀ of less than about 100 ng/mL, e.g., less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less, wherein preferably, the EC₅₀ is measured by ELISA;
(2) binding to ADAM9 (e.g., human or monkey ADAM9) with a KD of less than about 100 nM, e.g., less than about 90 nM, 80 nM, 70 nM, 60 nM, 50 nM, 40 nM, 30 nM, 20 nM, 15 nM, 10 nM, 5 nM or less, wherein preferably, the KD is measured by bio-layer interferometry (BLI) (such as ForteBio Octet^{®});
(3) having CDC activity, e.g., inducing killing of a cell expressing ADAM9 (e.g., a tumor cell) by CDC;
(4) having no ADCC activity;
(5) inducing ADAM9 internalization, e.g., as determined by flow cytometry;
(6) having no binding to ADAM12 and ADAM15 proteins;
(7) inhibiting the proliferation of a cell (e.g., a tumor cell); and/or
(8) inhibiting tumor growth.

14. An isolated nucleic acid molecule, comprising a nucleotide sequence encoding the antibody or the antigen-binding fragment thereof according to any one of claims 1 to 13, a heavy and/or light chain thereof, or a heavy and/or light chain variable region thereof.

15. The isolated nucleic acid molecule according to claim 14, comprising a nucleic acid molecule encoding the heavy chain variable region of the antibody, and/or a nucleic acid molecule encoding the light chain variable region of the antibody, wherein:
(a) the nucleic acid molecule encoding the heavy chain variable region of the antibody comprises: (i) a nucleotide sequence set forth in SEQ ID NO: 92, (ii) a sequence substantially identical to SEQ ID NO: 92 (e.g., a sequence having at least about 85%, 90%, 95%, 99% or higher sequence identity to SEQ ID NO: 92, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 93, (v) a sequence substantially identical to SEQ ID NO: 93 (e.g., a sequence having at least about 85%, 90%, 95%, 99% or higher sequence identity to SEQ ID NO: 93, or a sequence having one or more nucleotide substitutions), or (vi) a degenerate sequence of (iv) or (v) above;
or
(b) the nucleic acid molecule encoding the heavy chain variable region of the antibody comprises: (i) a nucleotide sequence set forth in SEQ ID NO: 44, (ii) a sequence substantially identical to SEQ ID NO: 44 (e.g., a sequence having at least about 85%, 90%, 95%, 99% or higher sequence identity to SEQ ID NO: 44, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 45, (v) a sequence substantially identical to SEQ ID NO: 45 (e.g., a sequence having at least about 85%, 90%, 95%, 99% or higher sequence identity to SEQ ID NO: 45, or a sequence having one or more nucleotide substitutions), or (vi) a degenerate sequence of (iv) or (v) above;
or
(c) the nucleic acid molecule encoding the heavy chain variable region of the antibody comprises: (i) a nucleotide sequence set forth in SEQ ID NO: 46, (ii) a sequence substantially identical to SEQ ID NO: 46 (e.g., a sequence having at least about 85%, 90%, 95%, 99% or higher sequence identity to SEQ ID NO: 46, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 47, (v) a sequence substantially identical to SEQ ID NO: 47 (e.g., a sequence having at least about 85%, 90%, 95%, 99% or higher sequence identity to SEQ ID NO: 47, or a sequence having one or more nucleotide substitutions), or (vi) a degenerate sequence of (iv) or (v) above;
or
(d) the nucleic acid molecule encoding the heavy chain variable region of the antibody comprises: (i) a nucleotide sequence set forth in SEQ ID NO: 90, (ii) a sequence substantially identical to SEQ ID NO: 90 (e.g., a sequence having at least about 85%, 90%, 95%, 99% or higher sequence identity to SEQ ID NO: 90, or a sequence having one or more nucleotide substitutions), or (iii) a degenerate sequence of (i) or (ii) above; and/or, the nucleic acid molecule encoding the light chain variable region of the antibody comprises: (iv) a nucleotide sequence set forth in SEQ ID NO: 91, (v) a sequence substantially identical to SEQ ID NO: 91 (e.g., a sequence having at least about 85%, 90%, 95%, 99% or higher sequence identity to SEQ ID NO: 91, or a sequence having one or more nucleotide substitutions), or (vi) a degenerate sequence of (iv) or (v) above.

16. A vector comprising the nucleic acid molecule according to either claim 14 or claim 15, wherein preferably, the vector is a cloning vector or an expression vector.

17. A host cell comprising the nucleic acid molecule according to either claim 14 or claim 15 or the vector according to claim 16.

18. A method for preparing the antibody or the antigen-binding fragment thereof according to any one of claims 1 to 13, comprising culturing the host cell according to claim 17 under conditions that allow expression of the antibody or the antigen-binding fragment thereof, and recovering the antibody or the antigen-binding fragment thereof from a culture of the cultured host cell.

19. A conjugate comprising the antibody or the antigen-binding fragment thereof according to any one of claims 1 to 13 and a conjugate moiety linked thereto,
wherein preferably, the conjugate moiety is selected from a detectable label (such as a radioisotope, a fluorescent substance, a luminescent substance, a colored substance or an enzyme) or a therapeutic agent (such as a cytotoxic agent, a cytokine, a toxin or a radionuclide).

20. A multispecific antibody comprising the antibody or the antigen-binding fragment thereof according to any one of claims 1 to 13,
wherein preferably, the multispecific antibody comprises the antibody or the antigen-binding fragment thereof according to any one of claims 1 to 13 as a first antigen-binding domain, and further comprises at least one second antigen-binding domain against other targets;
preferably, the multispecific antibody is a bispecific antibody or a trispecific antibody or a tetraspecific antibody.

21. A chimeric antigen receptor comprising the antibody or the antigen-binding fragment thereof (e.g., ScFv) according to any one of claims 1 to 13, a transmembrane domain, and one or more intracellular T cell signaling domains.

22. A pharmaceutical composition comprising the antibody or the antigen-binding fragment thereof according to any one of claims 1 to 13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor according to claim 21 or a host cell expressing the chimeric antigen receptor, and a pharmaceutically acceptable carrier and/or excipient,
wherein preferably, the pharmaceutical composition further comprises an additional pharmaceutically active agent;
preferably, the additional pharmaceutically active agent is a drug having anti-tumor activity;
preferably, the additional pharmaceutically active agent is selected from: an ADAM9 inhibitor, an EGFR inhibitor, an HER2 inhibitor, an HER3 inhibitor, an HER4 inhibitor, an IGFR-1 inhibitor, an mTOR inhibitor, a PI3 kinase inhibitor, a c-met inhibitor or a VEGF inhibitor, a chemotherapeutic agent or any combination thereof;
preferably, the antibody or the antigen-binding fragment thereof and the additional pharmaceutically active agent are provided as separate components or as a mixed component.

23. A diagnostic or therapeutic kit comprising the antibody or the antigen-binding fragment thereof according to any one of claims 1 to 13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor according to claim 21 or a host cell expressing the chimeric antigen receptor, or the pharmaceutical composition according to claim 22, and optionally instructions for use and/or an administration device.

24. Use of the antibody or the antigen-binding fragment thereof according to any one of claims 1 to 13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor according to claim 21 or a host cell expressing the chimeric antigen receptor, or the pharmaceutical composition according to claim 22 in the preparation of a drug for inhibiting the proliferation of a cell (e.g., a cell expressing ADAM9, e.g., a tumor cell), or preventing and/or treating and/or adjuvantly treating a tumor, a neurodegenerative disease, a retinal disease or inflammation,
wherein preferably, the antibody or the antigen-binding fragment thereof, the isolated nucleic acid molecule, the vector, the host cell, the conjugate, the multispecific antibody, or the pharmaceutical composition is administered in combination with an additional pharmaceutically active agent, e.g., simultaneously, separately, or sequentially;
preferably, the additional pharmaceutically active agent is a drug having anti-tumor activity;
preferably, the additional pharmaceutically active agent is selected from: an ADAM9 inhibitor, an EGFR inhibitor, an HER2 inhibitor, an HER3 inhibitor, an HER4 inhibitor, an IGFR-1 inhibitor, an mTOR inhibitor, a PI3 kinase inhibitor, a c-met inhibitor or a VEGF inhibitor, a chemotherapeutic agent or any combination thereof.

25. The use according to claim 24, wherein the tumor is an ADAM9-positive tumor;
preferably, the tumor is selected from glioma, cervical cancer, oral squamous cell carcinoma, liver cancer, melanoma, prostate cancer, pancreatic ductal adenocarcinoma, gastric cancer, lung cancer such as non-small cell lung cancer, pancreatic cancer, ovarian cancer, colorectal cancer, head and neck cancer, breast cancer such as triple-negative breast cancer, or any combination thereof.

26. A method for inhibiting the proliferation of a cell, comprising contacting the cell with the antibody or the antigen-binding fragment thereof according to any one of claims 1 to 13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor according to claim 21 or a host cell expressing the chimeric antigen receptor, or the pharmaceutical composition according to claim 22,
wherein preferably, the cell is a cell expressing ADAM9, e.g., a tumor cell;
preferably, the tumor cell overexpresses ADAM9.

27. A method for preventing and/or treating and/or adjuvantly treating a tumor, a neurodegenerative disease, a retinal disease or inflammation in a subject, comprising administering to a subject in need thereof an effective amount of the antibody or the antigen-binding fragment thereof according to any one of claims 1 to 13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor according to claim 21 or a host cell expressing the chimeric antigen receptor, or the pharmaceutical composition according to claim 22.

28. The method according to claim 27, further comprising administering to the subject a second therapy selected from surgery, chemotherapy, radiotherapy, immunotherapy, gene therapy, DNA therapy, RNA therapy, nanotherapy, viral therapy, adjuvant therapy and any combination thereof,
wherein optionally, the second therapy can be applied concurrently with, separately from, or sequentially to the method according to claim 27.

29. The method according to claim 27 or 28, wherein the tumor is an ADAM9-positive tumor; preferably, the tumor is selected from glioma, cervical cancer, oral squamous cell carcinoma, liver cancer, melanoma, prostate cancer, pancreatic ductal adenocarcinoma, gastric cancer, lung cancer such as non-small cell lung cancer, pancreatic cancer, ovarian cancer, colorectal cancer, head and neck cancer, breast cancer such as triple-negative breast cancer, or any combination thereof.

30. A method for detecting the presence or level of ADAM9 in a sample, comprising contacting the sample with the antibody or the antigen-binding fragment thereof according to any one of claims 1 to 13 under conditions that allow formation of a complex between the antibody or the antigen-binding fragment thereof and ADAM9, and detecting the formation of the complex,
wherein preferably, the method is used for diagnosing a tumor, e.g., an ADAM9-positive tumor, e.g., glioma, cervical cancer, oral squamous cell carcinoma, liver cancer, melanoma, prostate cancer, pancreatic ductal adenocarcinoma, gastric cancer, lung cancer such as non-small cell lung cancer, pancreatic cancer, ovarian cancer, colorectal cancer, head and neck cancer, breast cancer such as triple-negative breast cancer, or any combination thereof;
preferably, the method comprises detecting the expression level of ADAM9 in a test sample from a subject and comparing the expression level with a reference value, wherein an increase in the expression level compared to the reference value is an indication of a tumor.

31. Use of the antibody or the antigen-binding fragment thereof according to any one of claims 1-13, or the isolated nucleic acid molecule according to claim 14 or 15, or the vector according to claim 16, or the host cell according to claim 17, or the conjugate according to claim 19, or the multispecific antibody according to claim 20 in the preparation of a detection kit for detecting the presence or level of ADAM9 in a sample and/or diagnosing a tumor,
wherein preferably, the tumor is an ADAM9-positive tumor;
preferably, the tumor is selected from glioma, cervical cancer, oral squamous cell carcinoma, liver cancer, melanoma, prostate cancer, pancreatic ductal adenocarcinoma, gastric cancer, lung cancer such as non-small cell lung cancer, pancreatic cancer, ovarian cancer, colorectal cancer, head and neck cancer, breast cancer such as triple-negative breast cancer, or any combination thereof.
